# FASCICULE DE BREVET EUROPEEN

(11) **EP 3 083 537 B1**
(45) Date de publication et mention de la délivrance du brevet: **23.05.2018**
(21) Numéro de dépôt: 14827806.2
(22) Date de dépôt: 09.12.2014
(51) Int. Cl.: C07C 21/18, B01D 3/36

(54) **COMPOSITIONS AZEOTROPIQUES A BASE DE FLUORURE D'HYDROGENE ET DE Z-3,3,3-TRIFLUORO-1-CHLOROPROPENE**
AZEOTROPE ZUSAMMENSETZUNGEN AUS FLUORWASSERSTOFF UND Z-3,3,3-TRIFLUOR-1-CHLORPROPEN
AZEOTROPIC COMPOSITIONS OF HYDROGEN FLUORIDE AND Z-3,3,3-TRIFLUORO-1-CHLOROPROPENE

(30) Priorité: 19.12.2013 FR 1362982
(43) Date de publication de la demande: 26.10.2016
(73) Titulaire: Arkema France, 92700 Colombes (FR)
(72) Inventeur: DEUR-BERT, Dominique, F-69390 Charly (FR); PIGAMO, Anne, F-69340 Francheville (FR); BONNET, Philippe, F-69007 Lyon (FR)
(74) Mandataire: Leca, François Michel
(86) Numéro de dépôt international: PCT/FR2014/053228
(87) Numéro de publication internationale: WO 2015/092211

(56) Documents cités:
- US-B2- 8 075 797
- US-B2- 8 378 158

## Description

### DOMAINE DE L'INVENTION

La présente invention concerne des compositions azéotropiques ou quasi-azéotropiques à base de fluorure d'hydrogène et de Z-3,3,3-trifluoro-1-chloropropène.

### ARRIERE-PLAN TECHNIQUE

Le 3,3,3-trifluoro-1-chloropropène ou encore 1-chloro-3,3,3-trifluoropropène (HCFO-1233zd) existe sous forme de deux isomères: l'isomère cis, à savoir le Z-3,3,3-trifluoro-1-chloropropène (HCFO-1233zdZ), et l'isomère trans, à savoir le E-3,3,3-trifluoro-1-chloropropène (HCFO-1233zdE). Ils ont des points d'ébullition différents, respectivement de 18,5°C pour le composé trans et de 39,5°C pour le composé cis.

Les fluides à bases de E-3,3,3-trifluoro-1-chloropropène (HCFO-1233zdE) ont trouvé de nombreuses applications dans des domaines industriels variés, notamment en tant que fluides de transfert de chaleur, propulseurs, agents moussants, agents gonflants, diélectriques gazeux, milieux de polymérisation ou monomères, fluides supports, agents abrasifs, agents de séchage et fluides pour unité de production d'énergie.

La fabrication du HCFO-1233zdE s'accompagne d'une multitude de sous-produits, ayant un point d'ébullition proche du HCFO-1233zdE. Cela conduit à des étapes de purification assez complexes et coûteuses. Les difficultés rencontrées au cours de la purification du HCFO-1233zdE impliquent généralement une perte conséquente en produit recherché. De plus, les sous-produits peuvent former des compositions azéotropiques avec le HCFO-1233zdE, rendant très difficile voire impossible la séparation par distillation simple.

Le document US 6,013,846 décrit une composition azéotropique de HCFO-1233zd et de fluorure d'hydrogène (HF). Le document ne précise pas la forme isomérique du HCFO-1233zd.

Le document US 6,328,907 décrit une composition azéotropique de 1,1,1,3,3-pentafluoropropane (HFC-245fa) et de HF.

Le document US 8,378,158 décrit une composition quasi-azéotropique de HCFO-1233zdZ et de HF.

Le document US 7,423,188 décrit une composition azéotropique de E-1,3,3,3-tétrafluoropropène (HFO-1234zeE) et de HF.

Le document WO 2008/002500 décrit une composition azéotropique de Z-1,3,3,3-tétrafluoropropène (HFO-1234zeZ) et de HF.

Le document US 7,183,448 décrit une composition azéotropique de HFC-245fa et de HCFO-1233zd. Il est précisé dans le document que l'azéotrope est obtenu avec l'isomère trans du HCFO-1233zd.

Le document US 8,075,797 décrit une composition quasi-azéotropique de HF, de HFC-245fa et de HCFO-1233zd. Il est précisé dans le document que le quasi-azéotrope est obtenu avec l'isomère trans du HCFO-1233zd.

Il existe encore un besoin de fournir d'autres compositions azéotropiques et notamment des compositions azéotropiques à base de composés susceptibles d'intervenir dans la fabrication du HCFO-1233zdE. Or, de manière générale, les azéotropes sont difficilement prévisibles.

### RESUME DE L'INVENTION

L'invention concerne en premier lieu une composition azéotropique ou quasi-azéotropique comprenant du fluorure d'hydrogène, du Z-3,3,3-trifluoro-1-chloropropène et un ou plusieurs composés (hydro)halogénocarbonés comportant de 1 à 3 atomes de carbone.

Selon un mode de réalisation, le ou les composés (hydro)halogénocarbonés comportent 3 atomes de carbone, et sont de préférence choisis parmi les propanes et les propènes partiellement ou totalement substitués par des halogènes.

Selon un mode de réalisation, le ou les composés (hydro)halogénocarbonés sont choisis parmi les tétrachlorofluoropropanes, les trichlorodifluoropropanes, les dichlorotrifluoropropanes, les chlorotétrafluoropropanes, les pentafluoropropanes, les trichlorofluoropropènes, les dichlorodifluoropropènes, les chlorotrifluoropropènes et les tétrafluoropropènes.

Selon un mode de réalisation, la composition de l'invention comprend du fluorure d'hydrogène, du Z-3,3,3-trifluoro-1-chloropropène, du E-3,3,3-trifluoro-1-chloropropène et un ou plusieurs autres composés (hydro)halogénocarbonés comprenant de 1 à 3 atomes de carbone.

Selon un mode de réalisation, la composition de l'invention comprend du fluorure d'hydrogène, du Z-3,3,3-trifluoro-1-chloropropène, du E-1,3,3,3-tétrafluoropropène et un ou plusieurs autres composés (hydro)halogénocarbonés comprenant de 1 à 3 atomes de carbone.

Selon un mode de réalisation, la composition de l'invention comprend du fluorure d'hydrogène, du Z-3,3,3-trifluoro-1-chloropropène, du Z-1,3,3,3-tétrafluoropropène et un ou plusieurs autres composés (hydro)halogénocarbonés comprenant de 1 à 3 atomes de carbone.

Selon un mode de réalisation, la composition de l'invention comprend du fluorure d'hydrogène, du Z-3,3,3-trifluoro-1-chloropropène, du 1,1,1,3,3-pentafluoropropane et un ou plusieurs autres composés (hydro)halogénocarbonés comprenant de 1 à 3 atomes de carbone.

Selon un mode de réalisation, la composition de l'invention est un mélange ternaire, et de préférence est un mélange de fluorure d'hydrogène, de Z-3,3,3-trifluoro-1-chloropropène et de E-3,3,3-trifluoro-1-chloropropène.

Selon un mode de réalisation, la composition de l'invention est un mélange quaternaire, et de préférence est un mélange :
- de fluorure d'hydrogène, de Z-3,3,3-trifluoro-1-chloropropène, de E-3,3,3-trifluoro-1-chloropropène et de 1,1,1,3,3-pentafluoropropane ; ou
- de fluorure d'hydrogène, de Z-3,3,3-trifluoro-1-chloropropène, de E-1,3,3,3-tétrafluoropropène et de 1,1,1,3,3-pentafluoropropane ; ou
- de fluorure d'hydrogène, de Z-3,3,3-trifluoro-1-chloropropène, de Z-1,3,3,3-tétrafluoropropène et de 1,1,1,3,3-pentafluoropropane.

Selon un mode de réalisation, la composition de l'invention est un mélange quinquénaire, et de préférence est un mélange :
- de fluorure d'hydrogène, de Z-3,3,3-trifluoro-1-chloropropène, de E-3,3,3-trifluoro-1-chloropropène, de Z 1,3,3,3-tétrafluoropropène et de 1,1,1,3,3-pentafluoropropane ; ou
- de fluorure d'hydrogène, de Z-3,3,3-trifluoro-1-chloropropène, de E-3,3,3-trifluoro-1-chloropropène, de E 1,3,3,3-tétrafluoropropène et de 1,1,1,3,3-pentafluoropropane ; ou
- de fluorure d'hydrogène, de Z-3,3,3-trifluoro-1-chloropropène, de Z-1,3,3,3-tétrafluoropropène, de E-1,3,3,3-tétrafluoropropène et de 1,1,1,3,3-pentafluoropropane.

Selon un mode de réalisation, la composition de l'invention est un mélange sénaire, et de préférence est un mélange :
- de fluorure d'hydrogène, de Z-3,3,3-trifluoro-1-chloropropène, de E-3,3,3-trifluoro-1-chloropropène, de Z-1,3,3,3-tétrafluoropropène, de E-1,3,3,3-tétrafluoropropène et de 1,1,1,3,3-pentafluoropropane.

Selon un mode de réalisation, la composition de l'invention est hétéro-azéotropique ou quasi-hétéro-azéotropique.

Selon un mode de réalisation, la composition de l'invention comprend de 1 à 85 % en poids, de préférence de 1 à 80 % en poids, de manière plus particulièrement préférée de 5 à 80 % en poids et de manière tout particulièrement préférée de 5 à 75 % en poids de fluorure d'hydrogène ; et/ou de 15 à 99 % en poids, de préférence de 20 à 99 % en poids, de manière plus particulièrement préférée de 20 à 95 % en poids et de manière tout particulièrement préférée de 25 à 95 % en poids de composés (hydro)halogénocarbonés comportant de 1 à 3 atomes de carbone.

Selon un mode de réalisation, la composition de l'invention a un point d'ébullition de 0 à 40°C pour une pression de 0,5 à 9 bars absolu.

L'invention concerne également un procédé de production d'un composé (hydro)halogénocarboné principal, comprenant :
- la formation d'un mélange de composés comprenant du fluorure d'hydrogène, du Z-3,3,3-trifluoro-1-chloropropène et un ou plusieurs autres composés (hydro)halogénocarbonés ;
- la distillation de ce mélange, permettant de collecter d'une part une composition azéotropique de l'invention, et d'autre part au moins l'un des composés du mélange.

Selon un mode de réalisation, la distillation permet de collecter d'une part une composition azéotropique de l'invention, et d'autre part du fluorure d'hydrogène ; ou bien d'une part une composition azéotropique de l'invention, et d'autre part du E-3,3,3-trifluoro-1-chloropropène.

Selon un mode de réalisation, le procédé de l'invention est un procédé de production de 3,3,3-trifluoro-1-chloropropène, et de préférence de E-3,3,3-trifluoro-1-chloropropène.

Selon un mode de réalisation, le mélange de composés est obtenu à la suite d'une étape de fluoration, comprenant la réaction d'un composé chloré avec du fluorure d'hydrogène.

Selon un mode de réalisation, la composition azéotropique collectée est séparée, de préférence par décantation, en deux fractions liquides non-miscibles, à savoir une fraction riche en fluorure d'hydrogène et une fraction pauvre en fluorure d'hydrogène, la fraction riche en fluorure d'hydrogène contenant une proportion de fluorure d'hydrogène plus élevée que la fraction pauvre en fluorure d'hydrogène ; et la fraction riche en fluorure d'hydrogène étant le cas échéant recyclée vers l'étape de fluoration. La fraction pauvre en fluorure d'hydrogène peut faire l'objet d'une distillation pour permettre de collecter d'une part une composition azéotropique de l'invention et d'autre part du E-3,3,3-trifluoro-1chloropropène.

Selon un mode de réalisation, le composé chloré de départ réagissant avec le fluorure d'hydrogène est le 1,1,1,3,3-pentachloropropane ou le 1,1,3,3-tétrachloropropène.

La présente invention permet de répondre au besoin exprimé ci-dessus. Elle fournit plus particulièrement des compositions azéotropiques ou quasi-azéotropiques à partir de composés susceptibles d'intervenir dans la fabrication de divers composés (hydro)halogénocarbonés, et notamment dans la fabrication du HCFO-1233zdE.

L'identification de ces compositions azéotropiques ou quasi-azéotropiques permet ainsi notamment d'améliorer l'efficacité et les performances de procédés de production de composés (hydro)halogénocarbonés, et notamment de production de HCFO-1233zdE.

Dans un mode de réalisation préféré, ces compositions sont hétéro-azéotropiques, c'est-à-dire sont des compositions dont le liquide condensé forme deux solutions non miscibles qui peuvent être séparées aisément, par exemple par décantation. Cela comporte un avantage considérable pour les opérations de purification envisagées.

### BREVE DESCRIPTION DES FIGURES

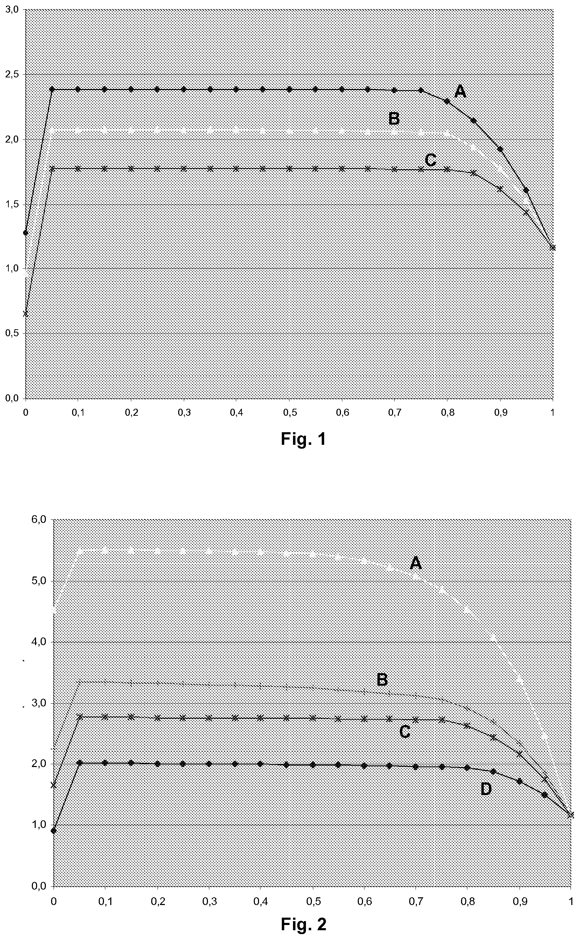

La **figure 1** représente la pression de vapeur (en ordonnée, en bars absolu) de mélanges selon l'invention conformes à l'exemple 1, pour l'isotherme de 25°C. En abscisse figure la fraction massique de HF dans la composition. Les courbes A, B et C correspondent à diverses compositions en composés (hydro)halogénocarbonés (voir l'exemple 1).
La **figure 2** représente la pression de vapeur (en ordonnée, en bars absolu) de mélanges selon l'invention conformes à l'exemple 2, pour l'isotherme de 25°C. En abscisse figure la fraction massique de HF dans la composition. Les courbes A, B, C et D correspondent à diverses compositions en composés (hydro)halogénocarbonés (voir l'exemple 2).

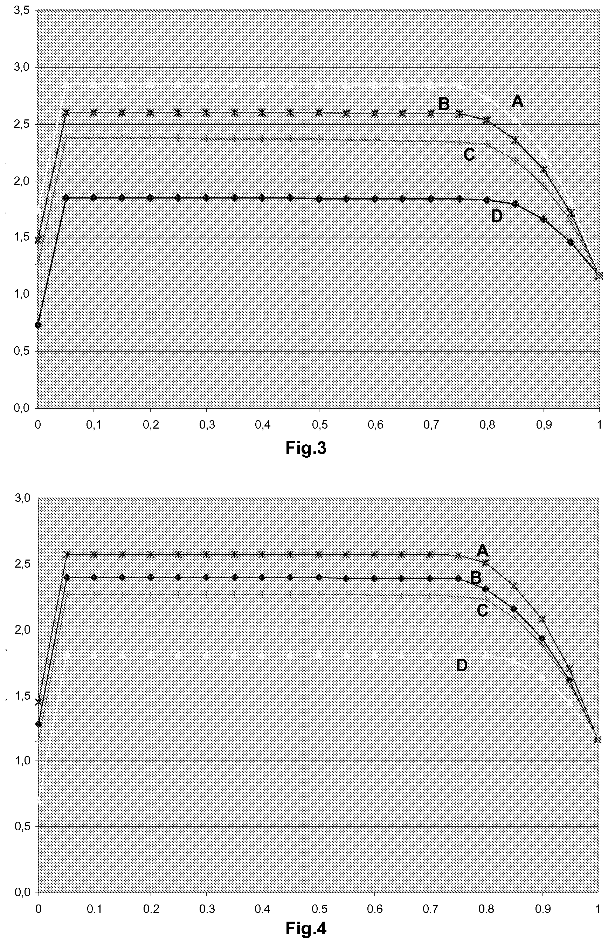

La **figure 3** représente la pression de vapeur (en ordonnée, en bars absolu) de mélanges selon l'invention conformes à l'exemple 3, pour l'isotherme de 25°C. En abscisse figure la fraction massique de HF dans la composition. Les courbes A, B, C et D correspondent à diverses compositions en composés (hydro)halogénocarbonés (voir l'exemple 3).
La **figure 4** représente la pression de vapeur (en ordonnée, en bars absolu) de mélanges selon l'invention conformes à l'exemple 4, pour l'isotherme de 25°C. En abscisse figure la fraction massique de HF dans la composition. Les courbes A, B, C et D correspondent à diverses compositions en composés (hydro)halogénocarbonés (voir l'exemple 4).

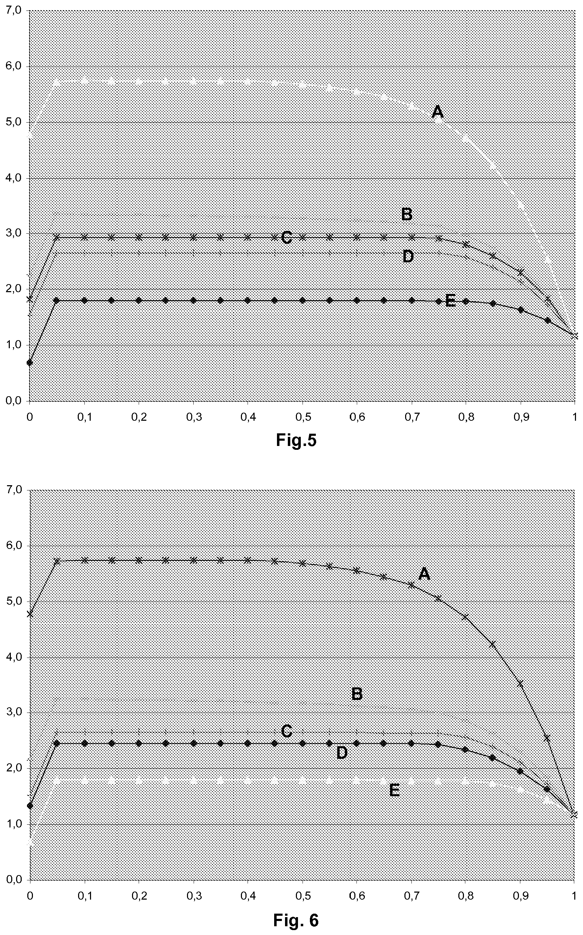

La **figure 5** représente la pression de vapeur (en ordonnée, en bars absolu) de mélanges selon l'invention conformes à l'exemple 5, pour l'isotherme de 25°C. En abscisse figure la fraction massique de HF dans la composition. Les courbes A, B, C, D et E correspondent à diverses compositions en composés (hydro)halogénocarbonés (voir l'exemple 5).
La **figure 6** représente la pression de vapeur (en ordonnée, en bars absolu) de mélanges selon l'invention conformes à l'exemple 6, pour l'isotherme de 25°C. En abscisse figure la fraction massique de HF dans la composition. Les courbes A, B, C, D et E correspondent à diverses compositions en composés (hydro)halogénocarbonés (voir l'exemple 6).

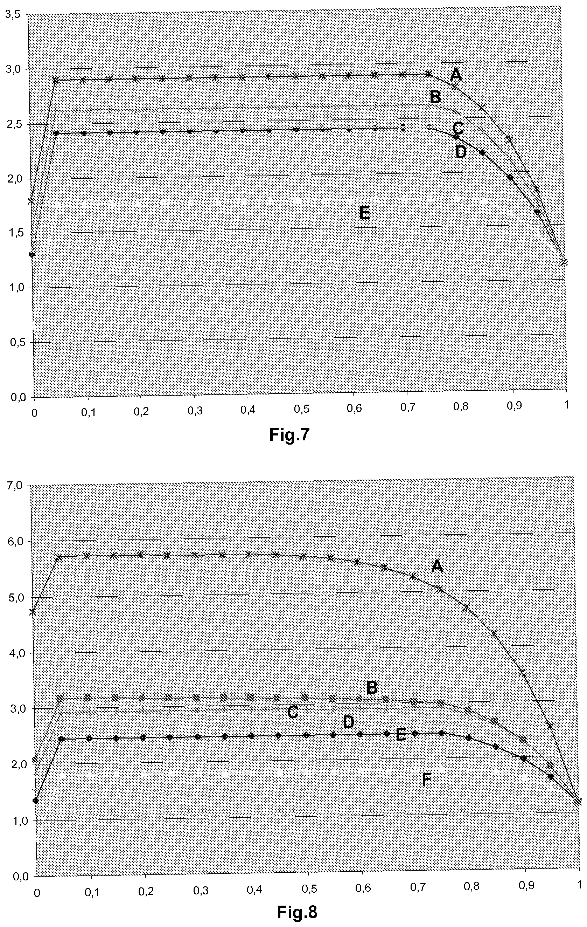

La **figure 7** représente la pression de vapeur (en ordonnée, en bars absolu) de mélanges selon l'invention conformes à l'exemple 7, pour l'isotherme de 25°C. En abscisse figure la fraction massique de HF dans la composition. Les courbes A, B, C, D et E correspondent à diverses compositions en composés (hydro)halogénocarbonés (voir l'exemple 7).
La **figure 8** représente la pression de vapeur (en ordonnée, en bars absolu) de mélanges selon l'invention conformes à l'exemple 8, pour l'isotherme de 25°C. En abscisse figure la fraction massique de HF dans la composition. Les courbes A, B, C, D, E et F correspondent à diverses compositions en composés (hydro)halogénocarbonés (voir l'exemple 8).

### DESCRIPTION DE MODES DE REALISATION DE L'INVENTION

L'invention est maintenant décrite plus en détail et de façon non limitative dans la description qui suit.

Sauf mention contraire toutes les proportions mentionnées dans la présente demande sont des proportions massiques.

L'invention fournit des compositions azéotropiques, quasi-azéotropiques, hétéro-azéotropiques et quasi-hétéro-azéotropiques.

Un mélange est considéré comme azéotropique lorsque que la pression au point de rosée est égale à celle au point de formation de bulle, ce qui signifie que la composition de la vapeur est égale à celle du liquide condensé.

Un mélange est considéré comme quasi-azéotropique lorsque la pression au point de rosée est substantiellement égale à celle au point de formation de bulle, ce qui signifie que la composition de vapeur est substantiellement égale à celle du liquide condensée : par exemple la différence de pression entre la pression au point de rosée et la pression au point de formation de bulle est inférieure ou égale à 5 %, sur la base de la pression au point de formation de bulles.

Un mélange hétéro-azéotropique est un mélange azéotropique dont le liquide condensé forme deux solutions non miscibles qui peuvent être facilement séparées, par exemple par décantation.

Un mélange quasi-hétéro-azéotropique est un mélange quasi-azéotropique dont le liquide condensé forme deux solutions non miscibles qui peuvent être facilement séparées, par exemple par décantation.

Les compositions selon l'invention comprennent du HF, du HCFO-1233zdZ et un ou plusieurs composés (hydro)halogénocarbonés comprenant de 1 à 3 atomes de carbone.

Les composés (hydro)halogénocarbonés sont des composés à base d'atomes de carbone, d'halogène(s) et optionnellement d'hydrogène ; avantageusement, ce sont des composés à base d'atomes de carbone, de chlore et/ou de fluor, et optionnellement d'hydrogène. Il s'agit avantageusement d'alcanes ou d'alcènes substitués partiellement ou totalement par des atomes d'halogènes, notamment chlore et/ou fluor.

Selon un mode de réalisation particulier, les composés (hydro)halogénocarbonés susceptibles d'être utilisés dans le cadre de l'invention comportent 1 ou 2 atomes de carbone.

Ils peuvent notamment être choisis parmi :
- le chlorométhane (HCC-40) ;
- le chloropentafluoroéthane (HCFC-115) ;
- le chlorotétrafluoroéthane (HFCF-124) à savoir le 1-chloro-1,2,2,2-tétrafluoroéthane et le 1-chloro-1,1,2,2-tétrafluoroéthane ;
- le pentafluoroéthane (HFC-125) ;
- le chlorotrifluoroéthane, à savoir en particulier le 1-chloro-1,2,2-trifluoroéthane (HCFC-133), le 1-chloro-2,2,2-trifluoroéthane (HCFC-133a) et le 1-chloro-1,1,2-trifluoroéthane (HCFC-133b) ;
- le tétrafluoroéthane, à savoir en particulier le 1,1,2,2-tétrafluoroéthane (HFC-134) et le 1,1,1,2-tétrafluoroéthane (HFC-134a) ;
- le chlorodifluoroéthane, à savoir en particulier le 1-chloro-2,2-difluoroéthane (HCFC-142), le 1-chloro-1,2-difluoroéthane (HCFC-142a) et le 1-chloro-1,1-difluoroéthane (HCFC-142b) ;
- le trifluoréthane, à savoir en particulier le 1,1,2-trifluoroéthane (HFC-143) et le 1,1,1-trifluoroéthane (HFC-143a) ;
- le difluoroéthane, à savoir en particulier le 1,1-difluoroéthane (HFC-152a) et le 1,2-difluoroéthane (HFC-152) ;
- le difluoroéthylène, à savoir le 1,2-difluoroéthylène (HFO-1132) et le 1,1-difluoroéthylène (HFO-1132a) ; et
- le fluoroéthylène (HFO-1141).

Selon un mode de réalisation particulier, les composés (hydro)halogénocarbonés susceptibles d'être utilisés dans le cadre de l'invention comportent 3 atomes de carbone.

Ils peuvent notamment être choisis parmi :
- le dichlorohexafluoropropane, à savoir en particulier le 1,2-dichloro-1,1,2,3,3,3-hexafluoropropane (HCFC-216ba), le 1,3-dichloro-1,1,2,2,3,3-hexafluoropropane (HCFC-216ca), le 1,1-dichloro-1,2,2,3,3,3-hexafluoropropane (HCFC-216cb) et le 2,2-dichloro-1,1,1,3,3,3-hexafluoropropane (HCFC-216aa);
- le chloroheptafluoropropane, à savoir en particulier le 1-chloro-1,1,2,2,3,3,3-heptafluoropropane (HCFC-217ca) et le 2-chloro-1,1,1,2,3,3,3-heptafluoropropane (HCFC-217ba) ;
- l'octafluoropropane (HFC-218) ;
- le dichloropentafluoropropane à savoir en particulier le 2,2-dichloro-1,1,1,3,3-pentafluoropropane (HCFC-225aa), le 2,3-dichloro-1,1,1,2,3-pentafluoropropane (HCFC-225ba), le 1,2-dichloro-1,1,2,3,3-pentafluoropropane (HCFC-225bb), le 3,3-dichloro-1,1,1,2,2-pentafluoropropane (HCFC-225ca), le 1,3-dichloro-1,1,2,2,3-pentafluoropropane (HCFC-225cb), le 1,1-dichloro-1,2,2,3,3-pentafluoropropane (HCFC-225cc), le 1,2-dichloro-1,1,3,3,3-pentafluoropropane (HCFC-225da), le 1,3-dichloro-1,1,2,3,3-pentafluoropropane (HCFC-225ea) et le 1,1-dichloro-1,2,3,3,3-pentafluoropropane (HCFC-225eb) ;
- le chlorohexafluoropropane, à savoir en particulier le 2-chloro-1,1,1,2,3,3-hexafluoropropane (HCFC-226ba), le 3-chloro-1,1,1,2,2,3-hexafluoropropane (HCFC-226ca), le 1-chloro-1,1,2,2,3,3-hexafluoropropane (HCFC-226cb), le 2-chloro-1,1,1,3,3,3-hexafluoropropane (HCFC-226da) et le 1-chloro-1,1,2,3,3,3-hexafluoropropane (HCFC-226ea) ;
- l'heptafluoropropane, à savoir en particulier le 1,1,2,2,3,3,3-heptafluoropropane (HFC-227ca) et le 1,1,1,2,3,3,3-heptafluoropropane (HFC-227ea) ;
- le dichlorotétrafluoropropane, à savoir en particulier le 2,2-dichloro-1,1,3,3-tétrafluoropropane (HCFC-234aa), le 2,2-dichloro-1,1,1,3-tétrafluoropropane (HCFC-234ab), le 1,2-dichloro-1,2,3,3-tétrafluoropropane (HCFC-234ba), le 2,3-dichloro-1,1,1,2-tétrafluoropropane (HCFC-234bb), le 1,2-dichloro-1,1,2,3-tétrafluoropropane (HCFC-234bc), le 1,3-dichloro-1,2,2,3-tétrafluoropropane (HCFC-234ca), le 1,1-dichloro-2,2,3,3-tétrafluoropropane (HCFC-234cb), le 1,3-dichloro-1,1,2,2-tétrafluoropropane (HCFC-234cc), le 1,1-dichloro-1,2,2,3-tétrafluoropropane (HCFC-234cd), le 2,3-dichloro-1,1,1,3-tétrafluoropropane (HCFC-234da), le 1,3-dichloro-1,1,3,3-tétrafluoropropane (HCFC-234fa), le 1,1-dichloro-1,3,3,3-tétrafluoropropane (HCFC-234fb), le 1,1-dichloro-2,3,3,3-tétrafluoropropane (HCFC-234ea), le 1,3-dichloro-1,1,2,3-tétrafluoropropane (HCFC-234eb) le 1,1-dichloro-1,2,3,3-tétrafluoropropane (HCFC-234ec) et le 1,2 dichloro-1,1,3,3-tétrafluoropentane (HCFC-234db);
- le chloropentafluoropropane, à savoir en particulier le 1-chloro-1,2,2,3,3-pentafluoropropane (HCFC-235ca), le 3-chloro-1,1,1,2,3-pentafluoropropane (HCFC-235ea), le 1-chloro-1,1,2,2,3-pentafluoropropane (HCFC-235cc), le 2-chloro-1,1,1,3,3-pentafluoropropane (HCFC-235da), le 1-chloro-1,1,3,3,3-pentafluoropropane (HCFC-235fa), le 1-chloro-1,1,2,3,3-pentafluoropropane (HCFC-235eb), le 3-chloro-1,1,1,2,2-pentafluoropropane (HCFC-235cb), le 2-chloro-1,1,2,3,3-pentafluoropropane (HCFC-235ba) et le 2-chloro-1,1,1,2,3-pentafluoropropane (HCFC-235bb) ;
- l'hexafluoropropane, à savoir en particulier le 1,1,1,2,2,3-hexafluoropropane (HFC-236cb), le 1,1,1,2,3,3-hexafluoropropane (HFC-236ea), le 1,1,1,3,3,3-hexafluoropropane (HFC-236fa) et le 1,1,2,2,3,3-hexafluoropropane (HFC-236ca) ;
- le tétrachlorofluoropropane, à savoir en particulier le 1,1,3,3-tétrachloro-1-fluoropropane (HCFC-241fa), le 1,1,1,3-tétrachloro-3-fluoropropane (HCFC-241fb), le 1,1,3,3-tétrachloro-2-fluoropropane (HCFC-241ea), le 1,1,1,3-tétrachloro-2-fluoropropane (HCFC-241eb), le 1,1,2,3-tétrachloro-3-fluoropropane (HCFC-241da), le 1,1,2,3-tétrachloro-1-fluoropropane (HCFC-241db), le 1,1,1,2-tétrachloro-3-fluoropropane (HCFC-241dc), le 1,1,2,3-tétrachloro-2-fluoropropane (HCFC-241ba), le 1,1,1,2-tétrchloro-2-fluoropropane (HCFC-241bb), le 1,2,2,3-tétrachloro-1-fluoropropane (HCFC-241aa), le 1,1,2,2-tétrachloro-3-fluoropropane (HCFC-241ab) et le 1,1,2,2-tétrachloro-1-fluoropropane (HCFC-241ac) ;
- le trichlorodifluoropropane, à savoir en particulier le 1,3,3-trichloro-1,1-difluoropropane (HCFC-242fa), le 1,1,3-trichloro-1,3-difluoropropane (HCFC-242fb), le 1,1,1,-trichloro-3,3-difluoropropane (HCFC-242fc), le 1,1,3-trichloro-2,3-difluoropropane (HCFC-242ea), le 1,1,3-trichloro-1,2-difluoropropane (HCFC-242eb), le 1,1,1-trichloro-2,3-difluoropropane (HCFC-242ec), le 1,2,3-trichloro-1,3-difluoropropane (HCFC-242da), le 1,1,2-trichloro-3,3-difluoropropane (HCFC-242db), le 1,2,3-trichloro-1,1-difluoropropane (HCFC-242dc), le 1,1,2-dichloro-1,3-difluoropropane (HCFC-242dd), le 1,1,3-trichloro-2,2-difluoropropane (HCFC-242ca), le 1,1,1-trichloro-2,2-difluoropropane (HCFC-242cb), le 1,2,3-trichloro-1,2-difluoropropane (HCFC-242ba), le 1,1,2-trichloro-2,3-difluoropropane (HCFC-242bb), le 1,1,2-trichloro-1,2-difluoropropane (HCFC-242bc), le 2,2,3-trichloro-1,1-difluoropropane (HCFC-242aa), le 1,2,2-trichloro-1,3-difluoropropane (HCFC-242ab) et le 1,2,2-trichloro-1,1-difluoropropane (HCFC-242ac) ;
- le dichlorotrifluoropropane, à savoir en particulier le 1,1-dichloro-3,3,3-trifluoropropane (HCFC-243fa), le 1,3-dichloro-1,1,3-trifluoropropane (HCFC-243fb), le 1,1-dichloro-1,3,3-trifluoropropane (HCFC-243fc), le 1,3-dichloro-1,2,3-trifluoropropane (HCFC-243ea), le 1,1-dichloro-2,3,3-trifluoropropane (HCFC-243eb), le 1,3-dichloro-1,1,2-trifluoropropane (HCFC-243ec), le 1,1-dichloro-1,2,3-trifluoropropane (HCFC-243ed), le 1,2-dichloro-1,3,3-trifluoropropane (HCFC-243da), le 2,3-dichloro-1,1,1-trifluoropropane (HCFC-243db), le 1,2-dichloro-1,1,3-trifluoropropane (HCFC-243dc), le 1,3-dichloro-1,2,2-trifluoropropane (HCFC-243ca), le 1,1-dichloro-2,2,3-trifluoropropane (HCFC-243cb), le 1,1-dichloro-1,2,2-trifluoropropane (HCFC-243cc), le 2,3-dichloro-1,1,2-trifluoropropane (HCFC-243ba), le 1,2-dichloro-1,2,3-trifluoropropane (HCFC-243bb), le 1,2-dichloro-1,1,2-trifluoropropane (HCFC-243bc), le 2,2-dichloro-1,1,3-trifluoropropane (HCFC-243aa) et le 2,2-dichloro-3,3,3-trifluoropropane (HCFC-243ab) ;
- le chlorotétrafluoropropane, à savoir en particulier le 2-chloro-1,2,3,3-tétrafluoropropane (HCFC-244ba), le 2-chloro-1,1,1,2-tétrafluoropropane (HCFC-244bb), le 3-chloro-1,1,2,2-tétrafluoropropane (HCFC-244ca), le 1-chloro-1,2,2,3-tétrafluoropropane (HCFC-244cb), le 1-chloro-1,1,2,2-tétrafluoropropane (HCFC-244cc), le 2-chloro-1,1,3,3-tétrafluoropropane (HCFC-244da), le 2-chloro-1,1,1,3-tétrafluoropropane (HCFC-244db), le 3-chloro-1,1,2,3-tétrafluoropropane (HCFC-244ea), le 3-chloro-1,1,1,2-tétrafluoropropane (HCFC-244eb), le 1-chloro-1,1,2,3-tétrafluoropropane (HCFC-244ec), le 3-chloro-1,1,1,3-tétrafluoropropane (HCFC-244fa) et le 1-chloro-1,1,3,3-tétrafluoropropane (HCFC-244fb) ;
- le pentafluoropropane, à savoir en particulier le 1,1,2,2,3-pentafluoropropane (HFC-245ca), le 1,1,2,3,3-pentafluoropropane (HFC-245ea), le 1,1,1,2,3-pentafluoropropane (HFC-245eb), le 1,1,1,2,2-pentafluoropropane (HFC-245cb) et le 1,1,1,3,3-pentafluoropropane (HFC-245fa) ;
- le chlorotrifluoropropane, à savoir en particulier le 2-chloro-1,2,3-trifluoropropane (HCFC-253ba), le 2-chloro-1,1,2-trifluoropropane (HCFC-253bb), le 1-chloro-2,2,3-trifluoropropane (HCFC-253ca), le 1-chloro-1,2,2-trifluoropropane (HCFC-253cb), le 3-chloro-1,1,2-trifluoropropane (HCFC-253ea), le 1-chloro-1,2,3-trifluoropropane (HCFC-253eb), le 1-chloro-1,1,2-trifluoropropane (HCFC-253ec), le 1-chloro-1,3,3-trifluoropropane (HCFC-253fa), le 3-chloro-1,1,1-trifluoropropane (HCFC-253fb), le 1-chloro-1,1,3-trifluoropropane (HCFC-253fc), le 2-chloro-1,1,3-trifluoropropane (HCFC-253da) et le 2-chloro-1,1,1-trifluoropropane (HCFC-253db) ;
- le tétrafluoropropane, à savoir en particulier le 1,1,2,2-tétrafluoropropane (HFC-254cb), le 1,1,1,3-tétrafluoropropane (HFC-254fb), le 1,1,2,3-tétrafluoropropane (HFC-254ea), le 1,1,1,2-tétrafluoropropane (HFC-254eb), le 1,2,2,3-tétrafluoropropane (HFC-254ca) et le 1,1,3,3-tétrafluoropropane (HFC-254fa);
- le chlorodifluoropropane, à savoir en particulier le 1-chloro-2,2-difluoropropane (HCFC-262ca), le 3-chloro-1,1-difluoropropane (HCFC-262fa), le 1-chloro-1,3-difluoropropane (HCFC-262fb), le 1-chloro-1,1-difluoropropane (HCFC-262fc), le 1-chloro-2,3-difluoropropane (HCFC-262ea), le 1-chloro-1,2-difluoropropane (HCFC-262eb), le 2-chloro-1,3-difluoropropane (HCFC-262da), le 2-chloro-1,1-difluoropropane (HCFC-262db) et le 2-chloro-1,2-difluoropropane (HCFC-262ba);
- le trifluoropropane (HFC-263), à savoir en particulier le 1,1,1-trifluoropropane (HFC-263fb), le 1,1,3-trifluoropropane (HFC-263fa), le 1,2,3-trifluoropropane (HFC-263ea), le 1,1,2-trifluoropropane (HFC-263eb) et le 1,2,2-trifluoropropane (HFC-263ca) ;
- le dichlorotétrafluoropropène (HCFO-1214), à savoir en particulier le 1,2-dichloro-1,3,3,3-tétrafluoropropène (HCFO-1214xb), le 1,1-dichloro-2,3,3,3-tétrafluoropropène (HCFO-1214ya), le 1,3-dichloro-1,2,3,3-tétrafluoropropène (HCFO-1214yb), le 2,3-dichloro-1,1,3,3-tétrafluoropropène (HCFO-1214xc) et le 3,3-dichloro-1,1,2,3-tétrafluoropropène (HCFO-1214yc);
- le chloropentafluoropropène (HCFO-1215), à savoir en particulier le 1-chloropentafluoropropène, le 2-chloropentafluoropropène et le 3-chloropentafluoropropène ;
- l'hexafluoropropène (HFO-1216) ;
- le dichlorotrifluoropropène (HCFO-1223), à savoir en particulier le 1,1-dichloro-3,3,3-trifluoropropène (HCFO-1223za), le 1,2-dichloro-3,3,3-trifluoropropène (HCFO-1223xd), le 2,3-dichloro-1,3,3-trifluoropropène (HCFO-1223xe), le 1,3-dichloro-2,3,3-trifluoropropène (HCFO-1223yd), le 1,2-dichloro-1,3,3-trifluoropropène (HCFO-1223xb), le 2,3-dichloro-1,1,3-trifluoropropène (HCFO-1223xc), le 1,1-dichloro-2,3,3-trifluoropropène (HCFO-1223ya), le 1,3-dichloro-1,2,3-trifluoropropène (HCFO-1223yb), le 3,3-dichloro-1,1,2-trifluoropropène (HCFO-1223yc), le 3,3-dichloro-1,2,3-trifluoropropène (HCFO-1223ye), le 1,3-dichloro-1,3,3-trifluoropropène (HCFO-1223zb) et le 3,3-dichloro-1,1,3-trifluoropropène (HCFO-1223zc);
- le chlorotétrafluoropropène (HCFO-1224), à savoir en particulier le 1-chloro-2,3,3,3-tetrafluoropropène (HCFO-1224yd), le 1-chloro-1,3,3,3-tetrafluoropropène (HCFO-1224zb), le 2-chloro-1,3,3,3-tetrafluoropropène (HCFO-1224xe), le 3-chloro-1,2,3,3-tetrafluoropropène (HCFO-1224ye), le 3-chloro-1,1,3,3-tetrafluoropropène (HCFO-1224zc), le 2-chloro-1,1,3,3-tetrafluoropropène (HCFO-1224xc), le 1-chloro-1,2,3,3-tetrafluoropropène (HCFO-1224yb) et le 3-chloro-1,1,2,3-tetrafluoropropène (HCFO-1224yc);
- le pentafluoropropène, à savoir en particulier le 1,1,1,2,3-pentafluoropropène sous forme trans (HFO-1225yeE), le 1,1,1,2,3-pentafluoropropène sous forme cis (HFO-1225yeZ), le 1,1,3,3,3-pentafluoropropène (HFO-1225zc) et le 1,1,2,3,3-pentafluoropropène (HFO-1225yc) ;
- le trichlorofluoropropène (HCFO-1231), à savoir en particulier le 1,1,2-trichloro-3-fluoropropène (HCFO-1231xa), le 1,2,3-trichloro-1-fluoropropène (HCFO-1231xb), le 1,2,3-trichloro-3-fluoropropène (HCFO-1231xd), le 2,3,3-trichloro-1-fluoropropène (HCFO-1231xe), le 2,3,3-trichloro-3-fluoropropène (HCFO-1231xf), le 1,1,3-trichloro-2-fluoropropène (HCFO-1231ya), le 1,3,3-trichloro-2-fluoropropène (HCFO-1231yd), 3,3,3-trichloro-2-fluoropropène (HCFO-1231yf), le 1,1,3-trichloro-3-fluoropropène (HCFO-1231za), le 1,3,3-trichloro-1-fluoropropène (HFCO-1231zb), 1,3,3-trichloro-3-fluoropropène (HCFO-1231zd) et le 3,3,3-trichloro-1-fluoropropène (HCFO-1231ze) ;
- le dichlorodifluoropropène (HCFO-1232), à savoir en particulier le 2,3-dichloro-3,3-difluoropropène (HCFO-1232xf), le 1,2-dichloro-1,3-difluoropropène (HCFO-1232xb), le 2,3-dichloro-1,1-difluoropropène (HCFO-1232xc), le 1,2-dichloro-3,3-difluoropropène (HCFO-1232xd), le 2,3-dichloro-1,3-difluoropropène (HCFO-1232xe), le 1,1-dichloro-2,3-difluoropropène (HCFO-1232ya), le 1,3-dichloro-1,2-difluoropropène (HCFO-1232yb), le 1,3-dichloro-2,3-difluoropropène (HCFO-1232yd), le 3,3-dichloro-1,2-difluoropropène (HCFO-1232ye), le 3,3-dichloro-2,3-difluoropropène (HCFO-1232yf), le 1,1-dichloro-3,3-difluoropropène (HCFO-1232za), le 1,3-dichloro-1,3-difluoropropène (HCFO-1232zb), le 3,3-dichloro-1,1-difluoropropène (HCFO-1232zc), le 1,3-dichloro-3,3-difluoropropène (HCFO-1232zd) et le 3,3-dichloro-1,3-difluoropropène (HCFO-1232ze) ;
- le chlorotrifluoropropène, à savoir en particulier le 2-chloro-1,1,3-trifluoropropène (HCFO-1233xc), le 2-chloro-1,3,3-trifluoropropène (HCFO-1233xe), le 1-chloro-1,2,3-trifluoropropène (HCFO-1233yb), le 3-chloro-1,1,2-trifluoropropène (HCFO-1233yc), le 1-chloro-2,3,3-trifluoropropène (HCFO-1233yd), le 3-chloro-1,2,3-trifluoropropène (HCFO-1233ye), le 3-chloro-2,3,3-trifluoropropène (HCFO-1233yf), le 1-chloro-1,3,3-trifluoropropène (HCFO-1233zb), le 3-chloro-1,1,3-trifluoropropène (HCFO-1233zc), le 3-chloro-1,3,3-trifluoropropène (HCFO-1233ze), le 2-chloro-3,3,3-trifluoro-propène (HCFO-1233xf) et le 1-chloro-3,3,3-trifuloropropène sous forme trans (HCFO-1233zdE) ;
- le tétrafluoropropène, à savoir en particulier le 1,1,2,3-tétrafluoropropène (HFO-1234yc), le 2,3,3,3-tétrafluoropropène (HFO-1234yf), le 1,2,3,3-tétrafluoropropène (HFO-1234ye), le 1,1,3,3-tétrafluoropropène (HFO-1234zc), le 1,3,3,3-tétrafluoropropène sous forme cis (HFO-1234zeZ) et le 1,3,3,3-tétrafluoropropène sous forme trans (HFO-1234zeE) ;
- le chlorodifluoropropène (HCFO-1242), à savoir en particulier le 3-chloro-3,3-difluoropropène (HCFO-1242zf), le 3-chloro-1,3-difluoropropène (HCFO-1242ze), le 2-chloro-1,1-difluoropropène (HCFO-1242xc), le 2-chloro-1,3-difluoropropène (HCFO-1242xe), le 2-chloro-3,3-difluoropropène (HCFO-1242xf), le 1-chloro-1,2-difluoropropène (HCFO-1242yb), le 1-chloro-2,3-difluoropropène (HCFO-1242yd), le 3-chloro-1,2-difluoropropène (HCFO-1242ye), le 3-chloro-2,3-difluoropropène (HCFO-1242yf), le 1-chloro-1,3-difluoropropène (HCFO-1242zb), le 3-chloro-1,1-difluoropropène (HCFO-1242zc) et le 1-chloro-3,3-difluoropropène (HCFO-1242zd) ;
- le trifluoropropène, à savoir en particulier le 1,1,2-trifluoropropène (HFO-1243yc), le 1,2,3-trifluoropropène (HFO-1243ye), le 2,3,3-trifluoropropène (HFO-1243yf), le 1,1,3-trifluoropropène (HFO-1243zc), le 1,3,3-trifluoropropène (HFO-1243ze) et le 3,3,3-trifluoropropène (HFO-1243zf) ;
- le chlorofluoropropène (HCFO-1251), à savoir en particulier le 1-chloro-3-fluoropropène (HCFO-1251zd), le 1-chloro-1-fluoropropène (HCFO-1251zb), le 1-chloro-2-fluoropropène (HCFO-1251yd), le 2-chloro-1-fluoropropène (HCFO-1251xe), le 2-chloro-3-fluoropropène (HCFO-1251 yf), le 3-chloro-2-fluoropropène (HCFO-1251xf), le 3-chloro-1-fluoropropène (HCFO-1251ze et le 3-chloro-3-fluoropropène (HCFO-1251 zf);
- le difluoropropène (HFO-1252), à savoir en particulier le 1,2-difluoropropène (HCFO-1252ye), le 2,3-difluoropropène (HCFO-1252yf), le 1,1-difluoropropène (HCFO-1252zc), le 1,3-difluoropropène (HCFO-1252ze) et le 3,3-difluoropropène (HCFO-1252zf); et
- le trifluoropropyne.

Les compositions selon l'invention peuvent être des mélanges ternaires, c'est-à-dire des mélanges de trois composés et trois seulement.

Les compositions selon l'invention peuvent être des mélanges quaternaires, c'est-à-dire des mélanges de quatre composés et quatre seulement.

Les compositions selon l'invention peuvent être des mélanges quinquénaires, c'est-à-dire des mélanges de cinq composés et cinq seulement.

Les compositions selon l'invention peuvent être des mélanges sénaires, c'est-à-dire des mélanges de six composés et six seulement.

Les compositions selon l'invention peuvent encore être des mélanges de sept composés et sept seulement.

Les compositions selon l'invention peuvent encore être des mélanges de huit composés et huit seulement.

Les compositions selon l'invention peuvent encore être des mélanges comprenant plus de huit composés.

Selon un mode de réalisation, les compositions selon l'invention consistent essentiellement en un mélange de HF, de HCFO-1233zdZ et d'un ou plusieurs composés (hydro)halogénocarbonés comprenant de 1 à 3 atomes de carbone - la quantité maximale d'impuretés, différentes de ces composés, étant par exemple de 2 %, ou de 1 %, ou de 0,5 %, ou de 0,2 %, ou de 0,1 %, ou de 0,05 %, ou de 0,02 % ou de 0,01 %.

Selon un mode de réalisation, les compositions selon l'invention consistent en un mélange de HF, de HCFO-1233zdZ et d'un ou plusieurs composés (hydro)halogénocarbonés comprenant de 1 à 3 atomes de carbone.

Selon un mode de réalisation, les composés (hydro)halogénocarbonés susmentionnés utilisés dans les compositions selon l'invention sont choisis parmi les tétrachlorofluoropropanes, les trichlorodifluoropropanes, les dichlorotrifluoropropanes, les chlorotétrafluoropropanes, les pentafluoropropanes, les dichlorodifluoropropènes, les trichlorofluoropropènes, les chlorotrifluoropropènes, les tétrafluoropropènes et leurs mélanges.

Selon un mode de réalisation, les composés (hydro)halogénocarbonés susmentionnés utilisés dans les compositions selon l'invention sont choisis parmi le HCFC-241fa, le HCFC-242fa, le HCFC-243fa, le HCFC-244fa, le HFC-245fa, le HCFO-1232za, le HCFO-1232zd, le HCFO-1233zdE, le HFO-1234zeZ et le HFO-1234zeE.

Selon un mode de réalisation, les composés (hydro)halogénocarbonés susmentionnés utilisés dans les compositions selon l'invention sont choisis parmi le HFC-245fa, le HCFO-1233zdE, le HFO-1234zeZ et le HFO-1234zeE.

Selon un mode de réalisation, les compositions selon l'invention peuvent consister (ou consister essentiellement en) un mélange de HF, de HCFO-1233zdE et de HCFO-1233zdZ. La teneur en HF dans ces compositions est avantageusement de 1 à 85 %, et plus préférentiellement de 5 à 80 %. Le point d'ébullition est de préférence de 0 à 40°C pour une pression de 0,6 à 4,0 bars absolu.

Alternativement les compositions selon l'invention peuvent comprendre un mélange de HF, de HCFO-1233zdE, de HCFO-1233zdZ et d'un ou plusieurs autres composés (hydro)halogénocarbonés, qui peuvent être choisis parmi l'ensemble des composés listés ci-dessus ; et qui peuvent être notamment choisis parmi les propanes et les propènes partiellement ou totalement substitués par des halogènes ; et qui peuvent être notamment choisis parmi les tétrachlorofluoropropanes, les trichlorodifluoropropanes, les dichlorotrifluoropropanes, les chlorotétrafluoropropanes, les pentafluoropropanes, les dichlorodifluoropropènes, les trichlorofluoropropènes, les chlorotrifluoropropènes et les tétrafluoropropènes ; et qui peuvent être notamment choisis parmi le HCFC-241fa, le HCFC-242fa, le HCFC-243fa, le HCFC-244fa, le HFC-245fa, le HCFO-1232za, le HCFO-1232zd, le HFO-1234zeZ et le HFO-1234zeE; et qui peuvent plus particulièrement être choisis parmi le HFC-245fa, le HFO-1234zeZ et le HFO-1234zeE.

Selon un mode de réalisation, les compositions selon l'invention peuvent consister (ou consister essentiellement en) un mélange de HF, de HCFO-1233zdE, de HCFO-1233zdZ et de HFC-245fa. La teneur en HF dans ces compositions est avantageusement de 1 à 85 %, et plus préférentiellement de 5 à 80 %. Le point d'ébullition est de préférence de 0 à 40°C pour une pression de 0,6 à 4,4 bars absolu.

Alternativement les compositions selon l'invention peuvent comprendre un mélange de HF, de HCFO-1233zdE, de HCFO-1233zdZ, de HFC-245fa et d'un ou plusieurs autres composés (hydro)halogénocarbonés, qui peuvent être choisis parmi l'ensemble des composés listés ci-dessus ; et qui peuvent être notamment choisis parmi les propanes et les propènes partiellement ou totalement substitués par des halogènes ; et qui peuvent être notamment choisis parmi les tétrachlorofluoropropanes, les trichlorodifluoropropanes, les dichlorotrifluoropropanes, les chlorotétrafluoropropanes, les pentafluoropropanes, les dichlorodifluoropropènes, les trichlorofluoropropènes, les chlorotrifluoropropènes et les tétrafluoropropènes ; et qui peuvent être notamment choisis parmi le HCFC-241fa, le HCFC-242fa, le HCFC-243fa, le HCFC-244fa, le HCFO-1232za, le HCFO-1232zd, le HFO-1234zeZ et le HFO-1234zeE; et qui peuvent plus particulièrement être choisis parmi le HFO-1234zeZ et le HFO-1234zeE.

Selon un mode de réalisation, les compositions selon l'invention peuvent consister (ou consister essentiellement en) un mélange de HF, de HCFO-1233zdZ, de HFO-1234zeZ et de HFC-245fa. La teneur en HF dans ces compositions est avantageusement de 1 à 85 %, et plus préférentiellement de 5 à 80 %. Le point d'ébullition est de préférence de 0 à 40°C pour une pression de 0,6 à 4,8 bars absolu.

Alternativement les compositions selon l'invention peuvent comprendre un mélange de HF, de HCFO-1233zdZ, de HFO-1234zeZ, de HFC-245fa et d'un ou plusieurs autres composés (hydro)halogénocarbonés, qui peuvent être choisis parmi l'ensemble des composés listés ci-dessus ; et qui peuvent être notamment choisis parmi les propanes et les propènes partiellement ou totalement substitués par des halogènes ; et qui peuvent être notamment choisis parmi les tétrachlorofluoropropanes, les trichlorodifluoropropanes, les dichlorotrifluoropropanes, les chlorotétrafluoropropanes, les pentafluoropropanes, les dichlorodifluoropropènes, les trichlorofluoropropènes, les chlorotrifluoropropènes et les tétrafluoropropènes ; et qui peuvent être notamment choisis parmi le HCFC-241fa, le HCFC-242fa, le HCFC-243fa, le HCFC-244fa, le HCFO-1232za, le HCFO-1232zd, le HCFO-1233zdE et le HFO-1234zeE; et qui peuvent plus particulièrement être choisis parmi le HCFO-1233zdE et le HFO-1234zeE.

Selon un mode de réalisation, les compositions selon l'invention peuvent consister (ou consister essentiellement en) un mélange de HF, de HCFO-1233zdZ, de HFO-1234zeE et de HFC-245fa. La teneur en HF dans ces compositions est avantageusement de 1 à 80 %, et plus préférentiellement de 5 à 75 %. Le point d'ébullition est de préférence de 0 à 40°C pour une pression de 0,6 à 8,6 bars absolu.

Alternativement les compositions selon l'invention peuvent comprendre un mélange de HF, de HCFO-1233zdZ, de HFO-1234zeE, de HFC-245fa et d'un ou plusieurs autres composés (hydro)halogénocarbonés, qui peuvent être choisis parmi l'ensemble des composés listés ci-dessus ; et qui peuvent être notamment choisis parmi les propanes et les propènes partiellement ou totalement substitués par des halogènes ; et qui peuvent être notamment choisis parmi les tétrachlorofluoropropanes, les trichlorodifluoropropanes, les dichlorotrifluoropropanes, les chlorotétrafluoropropanes, les pentafluoropropanes, les dichlorodifluoropropènes, les trichlorofluoropropènes, les chlorotrifluoropropènes et les tétrafluoropropènes ; et qui peuvent être notamment choisis parmi le HCFC-241fa, le HCFC-242fa, le HCFC-243fa, le HCFC-244fa, le HCFO-1232za, le HCFO-1232zd, le HCFO-1233zdE et le HFO-1234zeZ; et qui peuvent plus particulièrement être choisis parmi le HCFO-1233zdE et le HFO-1234zeZ.

Selon un mode de réalisation, les compositions selon l'invention peuvent consister (ou consister essentiellement en) un mélange de HF, de HCFO-1233zdZ, de HFO-1234zeE, de HFO-1234zeZ et de HFC-245fa. La teneur en HF dans ces compositions est avantageusement de 1 à 85 %, et plus préférentiellement de 5 à 80 %. Le point d'ébullition est de préférence de 0 à 40°C pour une pression de 0,6 à 8,9 bars absolu.

Alternativement les compositions selon l'invention peuvent comprendre un mélange de HF, de HCFO-1233zdZ, de HFO-1234zeE, de HFO-1234zeZ, de HFC-245fa et d'un ou plusieurs autres composés (hydro)halogénocarbonés, qui peuvent être choisis parmi l'ensemble des composés listés ci-dessus ; et qui peuvent être notamment choisis parmi les propanes et les propènes partiellement ou totalement substitués par des halogènes ; et qui peuvent être notamment choisis parmi les tétrachlorofluoropropanes, les trichlorodifluoropropanes, les dichlorotrifluoropropanes, les chlorotétrafluoropropanes, les pentafluoropropanes, les dichlorodifluoropropènes, les trichlorofluoropropènes, les chlorotrifluoropropènes et les tétrafluoropropènes ; et qui peuvent être notamment choisis parmi le HCFC-241fa, le HCFC-242fa, le HCFC-243fa, le HCFC-244fa, le HCFO-1232za, le HCFO-1232zd et le HCFO-1233zdE ; et qui peut notamment être le HCFO-1233zdE .

Selon un mode de réalisation, les compositions selon l'invention peuvent consister (ou consister essentiellement en) un mélange de HF, de HCFO-1233zdZ, de HCFO-1233zdE, de HFO-1234zeE et de HFC-245fa. La teneur en HF dans ces compositions est avantageusement de 1 à 85 %, et plus préférentiellement de 5 à 80 %. Le point d'ébullition est de préférence de 0 à 40°C pour une pression de 0,6 à 8,9 bars absolu.

Alternativement les compositions selon l'invention peuvent comprendre un mélange de HF, de HCFO-1233zdZ, de HCFO-1233zdE, de HFO-1234zeE, de HFC-245fa et d'un ou plusieurs autres composés (hydro)halogénocarbonés, qui peuvent être choisis parmi l'ensemble des composés listés ci-dessus ; et qui peuvent être notamment choisis parmi les propanes et les propènes partiellement ou totalement substitués par des halogènes ; et qui peuvent être notamment choisis parmi les tétrachlorofluoropropanes, les trichlorodifluoropropanes, les dichlorotrifluoropropanes, les chlorotétrafluoropropanes, les pentafluoropropanes, les dichlorodifluoropropènes, les trichlorofluoropropènes, les chlorotrifluoropropènes et les tétrafluoropropènes ; et qui peuvent être notamment choisis parmi le HCFC-241fa, le HCFC-242fa, le HCFC-243fa, le HCFC-244fa, le HCFO-1232za, le HCFO-1232zd et le HFO-1234zeZ ; et qui peut notamment être le HFO-1234zeZ.

Selon un mode de réalisation, les compositions selon l'invention peuvent consister (ou consister essentiellement en) un mélange de HF, de HCFO-1233zdZ, de HCFO-1233zdE, de HFO-1234zeZ et de HFC-245fa. La teneur en HF dans ces compositions est avantageusement de 1 à 85 %, et plus préférentiellement de 5 à 80 %. Le point d'ébullition est de préférence de 0 à 40°C pour une pression de 0,6 à 4,8 bars absolu.

Alternativement les compositions selon l'invention peuvent comprendre un mélange de HF, de HCFO-1233zdZ, de HCFO-1233zdE, de HFO-1234zeZ, de HFC-245fa et d'un ou plusieurs autres composés (hydro)halogénocarbonés, qui peuvent être choisis parmi l'ensemble des composés listés ci-dessus ; et qui peuvent être notamment choisis parmi les propanes et les propènes partiellement ou totalement substitués par des halogènes ; et qui peuvent être notamment choisis parmi les tétrachlorofluoropropanes, les trichlorodifluoropropanes, les dichlorotrifluoropropanes, les chlorotétrafluoropropanes, les pentafluoropropanes, les dichlorodifluoropropènes, les trichlorofluoropropènes, les chlorotrifluoropropènes et les tétrafluoropropènes ; et qui peuvent être notamment choisis parmi le HCFC-241fa, le HCFC-242fa, le HCFC-243fa, le HCFC-244fa, le HCFO-1232za, le HCFO-1232zd et le HFO-1234zeE ; et qui peut notamment être le HFO-1234zeE.

Selon un mode de réalisation, les compositions selon l'invention peuvent consister (ou consister essentiellement en) un mélange de HF, de HCFO-1233zdZ, de HCFO-1233zdE, de HFO-1234zeE, de HFO-1234zeZ, et de HFC-245fa. La teneur en HF dans ces compositions est avantageusement de 1 à 85 %, et plus préférentiellement de 5 à 80 %. Le point d'ébullition est de préférence de 0 à 40°C pour une pression de 0,6 à 8,8 bars absolu.

Alternativement les compositions selon l'invention peuvent comprendre un mélange de HF, de HCFO-1233zdZ, de HCFO-1233zdE, de HFO-1234zeE, de HFO-1234zeZ de HFC-245fa et d'un ou plusieurs autres composés (hydro)halogénocarbonés, qui peuvent être choisis parmi l'ensemble des composés listés ci-dessus ; et qui peuvent être notamment choisis parmi les propanes et les propènes partiellement ou totalement substitués par des halogènes ; et qui peuvent être notamment choisis parmi les tétrachlorofluoropropanes, les trichlorodifluoropropanes, les dichlorotrifluoropropanes, les chlorotétrafluoropropanes, les pentafluoropropanes, les dichlorodifluoropropènes, les trichlorofluoropropènes, les chlorotrifluoropropènes et les tétrafluoropropènes ; et qui peuvent être notamment choisis le HCFC 241fa, le HCFC-242fa, le HCFC-243fa, le HCFC-244fa, le HFO 1232za et le HCFO-1232zd.

De manière générale, dans les compositions selon l'invention, la proportion de HF est de préférence de 1 à 85 %, notamment de 5 à 80 % (par rapport au poids total de la composition), plus particulièrement de 10 à 65 % ; et la proportion de composés (hydro)halogénocarbonés, y compris le HCFO-1233zdZ, est de 15 à 99 %, notamment de 20 à 95 %, plus particulièrement de 35 à 90 %.

Le point d'ébullition d'une composition selon l'invention est de préférence de -20°C à 80°C pour une pression de 0,1 à 44 bars absolu ; avantageusement de 0 à 40°C pour une pression de 0,5 à 9 bars absolu.

Lorsque les compositions selon l'invention comprennent à la fois du HCFO-1233zdZ et du HCFO-1233zdE, celles-ci peuvent comprendre, en proportions massiques par rapport à la somme de ces deux composés :
- de 1 à 10 % de HCFO-1233zdZ et de 90 à 99 % de HCFO-1233zdE ;
- de 10 à 20 % de HCFO-1233zdZ et de 80 à 90 % de HCFO-1233zdE ;
- de 20 à 30 % de HCFO-1233zdZ et de 70 à 80 % de HCFO-1233zdE ;
- de 30 à 40 % de HCFO-1233zdZ et de 60 à 70 % de HCFO-1233zdE ;
- de 40 à 50 % de HCFO-1233zdZ et de 50 à 60 % de HCFO-1233zdE ;
- de 50 à 60 % de HCFO-1233zdZ et de 40 à 50 % de HCFO-1233zdE ;
- de 60 à 70 % de HCFO-1233zdZ et de 30 à 40 % de HCFO-1233zdE ;
- de 70 à 80 % de HCFO-1233zdZ et de 20 à 30 % de HCFO-1233zdE ;
- de 80 à 90 % de HCFO-1233zdZ et de 10 à 20 % de HCFO-1233zdE ;
- de 90 à 99 % de HCFO-1233zdZ et de 1 à 10 % de HCFO-1233zdE.

Selon un mode de réalisation, les compositions selon l'invention, à l'état condensé, comportent deux phases liquides, de préférence l'une plus riche en HF que l'autre.

L'invention peut en particulier être en particulier mise à profit dans le cadre de procédés de production d'un composé fluoré, dans lesquels des mélanges des composés décrits ci-dessus peuvent être produits.

De tels mélanges peuvent alors être traités par distillation afin de collecter d'une part une composition selon l'invention, et d'autre part du HF, ou bien d'autre part du HCFO-1233zdZ, ou bien d'autre part un ou plusieurs autres composés (hydro)halogénocarbonés.

De tels mélanges peuvent être notamment obtenus en tant que flux de produits à l'issue d'une réaction de fluoration catalytique d'un composé chloré en un composé fluoré par du HF.

Par « composé chloré » (qui représente le réactif principal de la réaction de fluoration catalytique), on entend un composé organique comprenant un ou plusieurs atomes de chlore, et par « composé fluoré » (qui représente le produit recherché de la réaction de fluoration catalytique), on entend un composé organique comprenant un ou plusieurs atomes de fluor.

Il est entendu que le composé chloré peut comprendre un ou plusieurs atomes de fluor, et que le composé fluoré peut comprendre un ou plusieurs atomes de chlore. De manière générale, le nombre d'atomes de chlore du composé fluoré est inférieur au nombre d'atomes de chlore du composé chloré ; et le nombre d'atomes de fluor du composé fluoré est supérieur au nombre d'atomes de fluor du composé chloré.

Le composé chloré peut être un alcane ou un alcène ayant éventuellement des substituants choisis parmi F, Cl, I et Br (de préférence parmi F et Cl), et comportant au moins un substituant Cl.

Le composé fluoré peut être un alcane ou un alcène ayant éventuellement des substituants choisis parmi F, Cl, I et Br (de préférence parmi F et Cl), et comportant au moins un substituant F.

Le composé chloré peut notamment être un alcane avec un ou plusieurs substituants chlore (hydrochlorocarbure ou chlorocarbure) ou un alcane avec un ou plusieurs substituants chlore et fluor (hydrochlorofluorocarbure ou chlorofluorocarbure) ou un alcène avec un ou plusieurs substituants chlore (chlorooléfine ou hydrochlorooléfine) ou un alcène avec un ou plusieurs substituants chlore et fluor (hydrochlorofluorooléfine ou chlorofluorooléfine).

Le composé fluoré peut notamment être un alcane avec un ou plusieurs substituants fluor (fluorocarbure ou hydrofluorocarbure) ou un alcane avec un ou plusieurs substituants chlore et fluor (hydrochlorofluorocarbure ou chlorofluorocarbure) ou un alcène avec un ou plusieurs substituants fluor (fluorooléfine ou hydrofluorooléfine) ou un alcène avec un ou plusieurs substituants chlore et fluor (hydrochlorofluorooléfine ou chlorofluorooléfine).

Selon un mode de réalisation, le composé chloré et le composé fluoré comportent un seul atome de carbone.

Selon un mode de réalisation, le composé chloré et le composé fluoré comportent deux atomes de carbone.

Selon un mode de réalisation particulièrement préféré, le composé chloré et le composé fluoré comportent trois atomes de carbone.

L'invention trouve notamment à s'appliquer pour les réactions de fluoration suivantes :
- fluoration du 1,1,1,3,3-pentachloropropane en HCFO-1233zdE ;
- fluoration du 1,1,3,3-tétrachloropropène en HCFO-1233zdE.

L'invention pourra s'appliquer notamment à la détermination des étapes de décantation et de purification nécessaires au traitement d'un flux gazeux sortant d'un réacteur de fluoration en phase liquide utilisant le 1,1,3,3-tétrachloropropène comme matière première ou bien d'un réacteur de fluoration en phase gaz utilisant le 1,1,1,3,3-pentachloropropane comme matière première. Ce flux gazeux peut contenir entre 15 et 50% de HF, entre 15 et 50% de HCl et le complément est constitué de l'ensemble des composés organiques issu de la réaction.

Dans un mode réalisation, on réalise un procédé de fluoration en phase liquide en utilisant le 1,1,3,3-tétrachloropropène comme matière première, et la répartition des composés organiques à l'issue de la réaction peut notamment être la suivante : de 85 à 95 % de HCFO-1233zdE, de 0 à 5% de HCFO-1233zdZ, de 0 à 3% de HFO-1234zeE, de 0 à 3% de HFO-1234zeZ, de 0 à 3 % de HFC-245fa, de 0 à 3% de HCFO-1232, de 0 à 1 % de HCFC-243 et de 0 à 1 % de HCFC-241.

Dans un mode de réalisation, on réalise un procédé de fluoration en phase gaz en utilisant le 1,1,1,3,3-pentachloropropane comme matière première, et la répartition des composés organiques à l'issue de la réaction peut notamment être la suivante : de 65 à 85% de HCFO-1233zdE, de 15 à 25% de HCFO-1233zdZ, de 0 à 10% de HCFO-1232, de 0 à 3% de HFO-1234zeE, de 0 à 3% de HFO-1234zeZ, de 0 à 3% de HFC-245fa, de 0 à 3% de HCFC-243.

Les compositions selon l'invention présentent des propriétés intéressantes en particulier pour le recyclage de l'HF à l'étape réactionnelle. Ainsi, la phase condensée de ces compositions, éventuellement lorsqu'elles sont soumises à une étape de distillation et/ou de une étape de séparation liquide/liquide, telle que par décantation, forme deux phases liquides non miscibles. La phase plus riche en HF peut être recyclée à l'étape réactionnelle, tandis que la phase moins riche en HF peut être soumise à une ou plusieurs étapes de distillation pour séparer les composés organiques et permettre par exemple de recycler à l'étape réactionnelle des composés organiques qui sont des intermédiaires réactionnels.

### EXEMPLES

Les exemples suivants illustrent l'invention sans la limiter.

### Exemple 1 - Composition ternaire HF / HCFO-1233zdE / HCFO-1233zdZ

Un comportement azéotropique et hétéro-azéotropique a été observé pour le mélange ternaire HF / HCFO-1233zdE / HCFO-1233zdZ.

La **figure** 1 illustre le comportement azéotropique pour l'isotherme à 25°C, pour diverses proportions HCFO-1233zdE / HCFO-1233zdZ, à savoir :
- A : 95 % de HCFO-1233zdE et 5 % HCFO-1233zdZ (par rapport au total des deux) ;
- B : 50 % de HCFO-1233zdE et 50 % HCFO-1233zdZ (par rapport au total des deux) ;
- C : 5 % de HCFO-1233zdE et 95 % HCFO-1233zdZ (par rapport au total des deux).

On constate que la composition a un point d'ébullition de 0 à 40°C pour une pression de 0,6 bars absolu à 4,0 bars absolu environ. Ainsi, pour un mélange de HF avec 50 % de HCFO-1233zdZ et 50 % de HCFO-1233zdE (les proportions étant par rapport à la somme des deux), le point d'ébullition est de 0°C à environ 0,9 bars absolu, de 25°C à environ 2,1 bars absolu et de 40°C à environ 3,5 bars absolu.

Les plages de décantation pour un mélange de HF avec 50 % de HCFO-1233zdZ et 50 % de HCFO-1233zdE (les proportions étant par rapport à la somme des deux) sont : de 5 à 75 % de HF à 0°C ; de 5 à 70 % de HF à 25°C; et de 5 à 60 % de HF à 40°C.

### Exemple 2 - Composition quaternaire HF / HCFO-1233zdZ / HFO-1234zeE / HFC-245fa

Un comportement azéotropique et hétéro-azéotropique a été observé pour le mélange quaternaire HF / HCFO-1233zdZ / HFO-1234zeE / HFC-245fa.

La **figure** 2 illustre le comportement azéotropique pour l'isotherme à 25°C, pour diverses proportions HCFO-1233zdZ / HFO-1234zeE / HFC-245fa, à savoir :
- A : 5 % de HCFO-1233zdZ, 90 % de HFO-1234zeE et 5 % de HFC-245fa (par rapport au total des trois) ;
- B : 40 % de HCFO-1233zdZ, 30 % de HFO-1234zeE et 30 % de HFC-245fa (par rapport au total des trois) ;
- C : 5 % de HCFO-1233zdZ, 5 % de HFO-1234zeE et 90 % de HFC-245fa (par rapport au total des trois) ;
- D : 90 % de HCFO-1233zdZ, 5 % de HFO-1234zeE et 5 % de HFC-245fa (par rapport au total des trois).

On constate que la composition a un point d'ébullition de 0 à 40°C pour une pression de 0,6 bars absolu à 8,6 bars absolu environ.

Les plages de décantation pour un mélange comprenant des proportions massiques égales de HCFO-1233zdZ, de HFO-1234zeE et de HFC-245fa sont : de 5 à 80 % de HF à 0°C ; de 5 à 75 % de HF à 25°C; et de 5 à 70 % de HF à 40°C.

### Exemple 3 - Composition quaternaire HF / HCFO-1233zdZ / HFO-1234zeZ / HFC-245fa

Un comportement azéotropique et hétéro-azéotropique a été observé pour le mélange quaternaire HF / HCFO-1233zdZ / HFO-1234zeZ / HFC-245fa.

La **figure 3** illustre le comportement azéotropique pour l'isotherme à 25°C, pour diverses proportions HCFO-1233zdZ / HFO-1234zeZ / HFC-245fa, à savoir :
- A : 5 % de HCFO-1233zdZ, 90 % de HFO-1234zeZ et 5 % de HFC-245fa (par rapport au total des trois) ;
- B : 5 % de HCFO-1233zdZ, 5 % de HFO-1234zeZ et 90 % de HFC-245fa (par rapport au total des trois) ;
- C : 40 % de HCFO-1233zdZ, 30 % de HFO-1234zeZ et 30 % de HFC-245fa (par rapport au total des trois) ;
- D : 90 % de HCFO-1233zdZ, 5 % de HFO-1234zeZ et 5 % de HFC-245fa (par rapport au total des trois).

On constate que la composition a un point d'ébullition de 0 à 40°C pour une pression de 0,6 bars absolu à 4,8 bars absolu environ.

Les plages de décantation pour un mélange comprenant des proportions massiques égales de HCFO-1233zdZ, de HFO-1234zeZ et de HFC-245fa sont : de 5 à 80 % de HF à 0°C ; de 5 à 75 % de HF à 25°C; et de 5 à 75 % de HF à 40°C.

### Exemple 4 - Composition quaternaire HF / HCFO-1233zdE / HCFO-1233zdZ / HFC-245fa

Un comportement azéotropique et hétéro-azéotropique a été observé pour le mélange quaternaire HF / HCFO-1233zdE / HCFO-1233zdZ / HFC-245fa.

La **figure 4** illustre le comportement azéotropique pour l'isotherme à 25°C, pour diverses proportions HCFO-1233zdE / HCFO-1233zdZ / HFC-245fa, à savoir :
- A : 5 % de HCFO-1233zdE, 5 % de HCFO-1233zdZ et 90 % de HFC-245fa (par rapport au total des trois) ;
- B : 90 % de HCFO-1233zdE, 5 % de HCFO-1233zdZ et 5 % de HFC-245fa (par rapport au total des trois) ;
- C : 40 % de HCFO-1233zdE, 30 % de HCFO-1233zdZ et 30 % de HFC-245fa (par rapport au total des trois) ;
- D : 5 % de HCFO-1233zdE, 90 % de HCFO-1233zdZ et 5 % de HFC-245fa (par rapport au total des trois).

On constate que la composition a un point d'ébullition de 0 à 40°C pour une pression de 0,6 bars absolu à 4,4 bars absolu environ.

Les plages de décantation pour un mélange comprenant des proportions massiques égales de HCFO-1233zdE, de HCFO-1233zdZ et de HFC-245fa sont : de 5 à 80 % de HF à 0°C ; de 5 à 75 % de HF à 25°C; et de 5 à 75 % de HF à 40°C.

### Exemple 5 - Composition quinquénaire HF / HCFO-1233zdZ / HFO-1234zeE / HFO-1234zeZ / HFC-245fa

Un comportement azéotropique et hétéro-azéotropique a été observé pour le mélange quinquénaire HF / HCFO-1233zdZ / HFO-1234zeE / HFO-1234zeZ / HFC-245fa.

La **figure 5** illustre le comportement azéotropique pour l'isotherme à 25°C, pour diverses proportions HCFO-1233zdZ / HFO-1234zeE / HFO-1234zeZ / HFC-245fa, à savoir :
- A : 1 % de HCFO-1233zdZ, 97 % de HFO-1234zeE, 1 % de HFO-1234zeZ et 1 % de HFC-245fa (par rapport au total des quatre) ;
- B : 25 % de HCFO-1233zdZ, 25 % de HFO-1234zeE, 25 % de HFO-1234zeZ et 25 % de HFC-245fa (par rapport au total des quatre) ;
- C : 1 % de HCFO-1233zdZ, 1 % de HFO-1234zeE, 97 % de HFO-1234zeZ et 1 % de HFC-245fa (par rapport au total des quatre) ;
- D : 1 % de HCFO-1233zdZ, 1 % de HFO-1234zeE, 1 % de HFO-1234zeZ et 97 % de HFC-245fa (par rapport au total des quatre) ;
- E : 97 % de HCFO-1233zdZ, 1 % de HFO-1234zeE, 1 % de HFO-1234zeZ et 1 % de HFC-245fa (par rapport au total des quatre).

On constate que la composition a un point d'ébullition de 0 à 40°C pour une pression de 0,6 bars absolu à 8,9 bars absolu environ.

Les plages de décantation pour un mélange comprenant des proportions massiques égales de HCFO-1233zdZ, de HFO-1234zeE, de HFO-1234zeZ et de HFC-245fa sont : de 5 à 80 % de HF à 0°C ; de 5 à 75 % de HF à 25°C ; et de 5 à 65 % de HF à 40°C.

### Exemple 6 - Composition quinquénaire HF / HCFO-1233zdE / HCFO-1233zdZ / HFO-1234zeE / HFC-245fa

Un comportement azéotropique et hétéro-azéotropique a été observé pour le mélange quinquénaire HF / HCFO-1233zdE / HCFO-1233zdZ / HFO-1234zeE / HFC-245fa.

La **figure 6** illustre le comportement azéotropique pour l'isotherme à 25°C, pour diverses proportions HCFO-1233zdE / HCFO-1233zdZ / HFO-1234zeE / HFC-245fa, à savoir :
- A: 1 % de HCFO-1233zdE, 1 % de HCFO-1233zdZ, 97 % de HFO-1234zeE et 1 % de HFC-245fa (par rapport au total des quatre) ;
- B : 25 % de HCFO-1233zdE, 25 % de HCFO-1233zdZ, 25 % de HFO-1234zeE et 25 % de HFC-245fa (par rapport au total des quatre) ;
- C : 1 % de HCFO-1233zdE, 1 % de HCFO-1233zdZ, 1 % de HFO-1234zeE et 97 % de HFC-245fa (par rapport au total des quatre) ;
- D : 97 % de HCFO-1233zdE, 1 % de HCFO-1233zdZ, 1 % de HFO-1234zeE et 1 % de HFC-245fa (par rapport au total des quatre) ;
- E : 1 % de HCFO-1233zdE, 97 % de HCFO-1233zdZ, 1 % de HFO-1234zeE et 1 % de HFC-245fa (par rapport au total des quatre).

On constate que la composition a un point d'ébullition de 0 à 40°C pour une pression de 0,6 bars absolu à 8,9 bars absolu environ.

Les plages de décantation pour un mélange comprenant des proportions massiques égales de HCFO-1233zdE, de HCFO-1233zdZ, de HFO-1234zeE et de HFC-245fa sont : de 5 à 80 % de HF à 0°C ; de 5 à 75 % de HF à 25°C; et de 5 à 65 % de HF à 40°C.

### Exemple 7 - Composition quinquénaire HF / HCFO-1233zdE / HCFO-1233zdZ / HFO-1234zeZ / HFC-245fa

Un comportement azéotropique et hétéro-azéotropique a été observé pour le mélange quinquénaire HF / HCFO-1233zdE / HCFO-1233zdZ / HFO-1234zeZ / HFC-245fa.

La **figure 7** illustre le comportement azéotropique pour l'isotherme à 25°C, pour diverses proportions HCFO-1233zdE / HCFO-1233zdZ / HFO-1234zeZ / HFC-245fa, à savoir :
- A: 1 % de HCFO-1233zdE, 1 % de HCFO-1233zdZ, 97 % de HFO-1234zeZ et 1 % de HFC-245fa (par rapport au total des quatre) ;
- B : 1 % de HCFO-1233zdE, 1 % de HCFO-1233zdZ, 1 % de HFO-1234zeZ et 97 % de HFC-245fa (par rapport au total des quatre) ;
- C : 25 % de HCFO-1233zdE, 25 % de HCFO-1233zdZ, 25 % de HFO-1234zeZ et 25 % de HFC-245fa (par rapport au total des quatre) ;
- D : 97 % de HCFO-1233zdE, 1 % de HCFO-1233zdZ, 1 % de HFO-1234zeZ et 1 % de HFC-245fa (par rapport au total des quatre) ;
- E : 1 % de HCFO-1233zdE, 97 % de HCFO-1233zdZ, 1 % de HFO-1234zeZ et 1 % de HFC-245fa (par rapport au total des quatre).

On constate que la composition a un point d'ébullition de 0 à 40°C pour une pression de 0,6 bars absolu à 4,8 bars absolu environ.

Les plages de décantation pour un mélange comprenant des proportions massiques égales de HCFO-1233zdE, de HCFO-1233zdZ, de HFO-1234zeZ et de HFC-245fa sont : de 5 à 80 % de HF à 0°C ; de 5 à 75 % de HF à 25°C; et de 5 à 70 % de HF à 40°C.

### Exemple 8 - Composition sénaire HF / HCFO-1233zdE / HCFO-1233zdZ / HFO-1234zeE / HFO-1234zeZ / HFC-245fa

Un comportement azéotropique et hétéro-azéotropique a été observé pour le mélange sénaire HF / HCFO-1233zdE / HCFO-1233zdZ / HFO-1234zeE / HFO-1234zeZ / HFC-245fa.

La **figure 8** illustre le comportement azéotropique pour l'isotherme à 25°C, pour diverses proportions HCFO-1233zdE / HCFO-1233zdZ / HFO-1234zeE / HFO-1234zeZ / HFC-245fa, à savoir :
- A: 1 % de HCFO-1233zdE, 1 % de HCFO-1233zdZ, 96 % de HFO-1234zeE, 1 % de HFO-1234zeZ et 1 % de HFC-245fa (par rapport au total des cinq) ;
- B : 20 % de HCFO-1233zdE, 20 % de HCFO-1233zdZ, 20 % de HFO-1234zeE, 20 % de HFO-1234zeZ et 20 % de HFC-245fa (par rapport au total des cinq) ;
- C : 1 % de HCFO-1233zdE, 1 % de HCFO-1233zdZ, 1 % de HFO-1234zeE, 96 % de HFO-1234zeZ et 1 % de HFC-245fa (par rapport au total des cinq) ;
- D : 1 % de HCFO-1233zdE, 1 % de HCFO-1233zdZ, 1 % de HFO-1234zeE, 1 % de HFO-1234zeZ et 96 % de HFC-245fa (par rapport au total des cinq) ;
- E : 96 % de HCFO-1233zdE, 1 % de HCFO-1233zdZ, 1 % de HFO-1234zeE, 1 % de HFO-1234zeZ et 1 % de HFC-245fa (par rapport au total des cinq) ;
- F : 1 % de HCFO-1233zdE, 96 % de HCFO-1233zdZ, 1 % de HFO-1234zeE, 1 % de HFO-1234zeZ et 1 % de HFC-245fa (par rapport au total des cinq).

On constate que la composition a un point d'ébullition de 0 à 40°C pour une pression de 0,6 bars absolu à 8,8 bars absolu environ.

Les plages de décantation pour un mélange comprenant des proportions massiques égales de HCFO-1233zdE, de HCFO-1233zdZ, de HFO-1234zeE, de HFO-1234zeZ et de HFC-245fa sont : de 5 à 80 % de HF à 0°C ; de 5 à 75 % de HF à 25°C ; et de 5 à 65 % de HF à 40°C.

## Revendications

1. Composition azéotropique ou quasi-azéotropique comprenant du fluorure d'hydrogène, du Z-3,3,3-trifluoro-1-chloropropène et un ou plusieurs composés (hydro)halogénocarbonés comportant de 1 à 3 atomes de carbone.

2. Composition selon la revendication 1, dans lequel le ou les composés (hydro)halogénocarbonés comportent 3 atomes de carbone, et sont de préférence choisis parmi les propanes et les propènes partiellement ou totalement substitués par des halogènes.

3. Composition selon la revendication 1 ou 2, dans laquelle le ou les composés (hydro)halogénocarbonés sont choisis parmi les tétrachlorofluoropropanes, les trichlorodifluoropropanes, les dichlorotrifluoropropanes, les chlorotétrafluoropropanes, les pentafluoropropanes, les dichlorodifluoropropènes, les trichlorofluoropropènes, les chlorotrifluoropropènes et les tétrafluoropropènes.

4. Composition selon l'une des revendications 1 à 3, comprenant du fluorure d'hydrogène, du Z-3,3,3-trifluoro-1-chloropropène, du E-3,3,3-trifluoro-1-chloropropène et un ou plusieurs autres composés (hydro)halogénocarbonés comprenant de 1 à 3 atomes de carbone.

5. Composition selon l'une des revendications 1 à 4, comprenant du fluorure d'hydrogène, du Z-3,3,3-trifluoro-1-chloropropène, du E-1,3,3,3-tétrafluoropropène et un ou plusieurs autres composés (hydro)halogénocarbonés comprenant de 1 à 3 atomes de carbone.

6. Composition selon l'une des revendications 1 à 5, comprenant du fluorure d'hydrogène, du Z-3,3,3-trifluoro-1-chloropropène, du Z-1,3,3,3-tétrafluoropropène et un ou plusieurs autres composés (hydro)halogénocarbonés comprenant de 1 à 3 atomes de carbone.

7. Composition selon l'une des revendications 1 à 6, comprenant du fluorure d'hydrogène, du Z-3,3,3-trifluoro-1-chloropropène, du 1,1,1,3,3-pentafluoropropane et un ou plusieurs autres composés (hydro)halogénocarbonés comprenant de 1 à 3 atomes de carbone.

8. Composition selon l'une des revendications 1 à 7, qui est un mélange ternaire, et de préférence est un mélange de fluorure d'hydrogène, de Z-3,3,3-trifluoro-1-chloropropène et de E-3,3,3-trifluoro-1-chloropropène.

9. Composition selon l'une des revendications 1 à 7, qui est un mélange quaternaire, et de préférence est un mélange :
- de fluorure d'hydrogène, de Z-3,3,3-trifluoro-1-chloropropène, de E-3,3,3-trifluoro-1-chloropropène et de 1,1,1,3,3-pentafluoropropane ; ou
- de fluorure d'hydrogène, de Z-3,3,3-trifluoro-1-chloropropène, de E-1,3,3,3-tétrafluoropropène et de 1,1,1,3,3-pentafluoropropane ; ou
- de fluorure d'hydrogène, de Z-3,3,3-trifluoro-1-chloropropène, de Z-1,3,3,3-tétrafluoropropène et de 1,1,1,3,3-pentafluoropropane.

10. Composition selon l'une des revendications 1 à 7, qui est un mélange quinquénaire, et de préférence est un mélange :
- de fluorure d'hydrogène, de Z-3,3,3-trifluoro-1-chloropropène, de E-3,3,3-trifluoro-1-chloropropène, de Z-1,3,3,3-tétrafluoropropène et de 1,1,1,3,3-pentafluoropropane ; ou
- de fluorure d'hydrogène, de Z-3,3,3-trifluoro-1-chloropropène, de E-3,3,3-trifluoro-1-chloropropène, de E-1,3,3,3-tétrafluoropropène et de 1,1,1,3,3-pentafluoropropane ; ou
- de fluorure d'hydrogène, de Z-3,3,3-trifluoro-1-chloropropène, de Z-1,3,3,3-tétrafluoropropène, de E-1,3,3,3-tétrafluoropropène et de 1,1,1,3,3-pentafluoropropane.

11. Composition selon l'une des revendications 1 à 7, qui est un mélange sénaire, et de préférence est un mélange :
- de fluorure d'hydrogène, de Z-3,3,3-trifluoro-1-chloropropène, de E-3,3,3-trifluoro-1-chloropropène, de Z-1,3,3,3-tétrafluoropropène, de E-1,3,3,3-tétrafluoropropène et de 1,1,1,3,3-pentafluoropropane.

12. Composition selon l'une des revendications 1 à 11, qui est hétéro-azéotropique ou quasi-hétéro-azéotropique.

13. Composition selon l'une des revendications 1 à 12, qui comprend de 1 à 85 % en poids, de préférence de 1 à 80 % en poids, de manière plus particulièrement préférée de 5 à 80 % en poids et de manière tout particulièrement préférée de 5 à 75 % en poids de fluorure d'hydrogène ; et/ou de 15 à 99 % en poids, de préférence de 20 à 99 % en poids, de manière plus particulièrement préférée de 20 à 95 % en poids et de manière tout particulièrement préférée de 25 à 95 % en poids de composés (hydro)halogénocarbonés comportant de 1 à 3 atomes de carbone.

14. Composition selon l'une des revendications 1 à 13, ayant un point d'ébullition de 0 à 40°C pour une pression de 0,5 à 9 bars absolu.

15. Procédé de production d'un composé (hydro)halogénocarboné principal, comprenant :
- la formation d'un mélange de composés comprenant du fluorure d'hydrogène, du Z-3,3,3-trifluoro-1-chloropropène et un ou plusieurs autres composés (hydro)halogénocarbonés ;
- la distillation de ce mélange, permettant de collecter d'une part une composition azéotropique selon l'une des revendications 1 à 14, et d'autre part au moins l'un des composés du mélange.

16. Procédé selon la revendication 15, dans lequel la distillation permet de collecter d'une part une composition azéotropique selon l'une des revendications 1 à 14, et d'autre part du fluorure d'hydrogène ; ou bien d'une part une composition azéotropique selon l'une des revendications 1 à 14, et d'autre part du E-3,3,3-trifluoro-1-chloropropène.

17. Procédé selon la revendication 15 ou 16, qui est un procédé de production de 3,3,3-trifluoro-1-chloropropène, et de préférence de E-3,3,3-trifluoro-1-chloropropène.

18. Procédé selon l'une des revendications 15 à 17, dans lequel le mélange de composés est obtenu à la suite d'une étape de fluoration, comprenant la réaction d'un composé chloré avec du fluorure d'hydrogène.

19. Procédé selon l'une des revendications 15 à 18, dans lequel la composition azéotropique collectée est séparée, de préférence par décantation, en deux fractions liquides non-miscibles, à savoir une fraction riche en fluorure d'hydrogène et une fraction pauvre en fluorure d'hydrogène, la fraction riche en fluorure d'hydrogène contenant une proportion de fluorure d'hydrogène plus élevée que la fraction pauvre en fluorure d'hydrogène ; et la fraction riche en fluorure d'hydrogène étant le cas échéant recyclée vers l'étape de fluoration.

20. Procédé selon l'une des revendications 15 à 19, dans lequel le composé chloré est le 1,1,1,3,3-pentachloropropane ou le 1,1,3,3-tétrachloropropène.

## Patentansprüche

1. Azeotrope oder quasi-azeotrope Zusammensetzung, umfassend Fluorwasserstoff, Z-3,3,3-Trifluor-1-chlorpropen und eine oder mehrere (Hydro)Halogenkohlenstoff-Verbindungen mit 1 bis 3 Kohlenstoffatomen.

2. Zusammensetzung nach Anspruch 1, wobei die (Hydro)Halogenkohlenstoff-Verbindung(en) 3 Kohlenstoffatome umfasst (umfassen) und vorzugsweise ausgewählt ist (sind) aus Propanen und Propenen, die teilweise oder vollständig durch Halogene substituiert sind.

3. Zusammensetzung nach Anspruch 1 oder 2, wobei die (Hydro)Halogenkohlenstoff-Verbindung (en) ausgewählt ist (sind) aus Tetrachlorfluorpropanen, Trichlordifluorpropanen, Dichlortrifluorpropanen, Chlortetrafluorpropanen, Pentafluorpropanen, Dichlordifluorpropenen, Trichlorfluorpropenen, Chlortrifluorpropenen und Tetrafluorpropenen.

4. Zusammensetzung nach einem der Ansprüche 1 bis 3, umfassend Fluorwasserstoff, Z-3,3,3-Trifluor-1-chlorpropen, E-3,3,3-Trifluor-1-chlorpropen und eine oder mehrere andere (Hydro)Halogenkohlenstoff-Verbindungen mit 1 bis 3 Kohlenstoffatomen.

5. Zusammensetzung nach einem der Ansprüche 1 bis 4, umfassend Fluorwasserstoff, Z-3,3,3-Trifluor-1-chlorpropen, E-1,3,3,3-Tetrafluorpropen und eine oder mehrere andere (Hydro)Halogenkohlenstoff-Verbindungen mit 1 bis 3 Kohlenstoffatomen.

6. Zusammensetzung nach einem der Ansprüche 1 bis 5, umfassend Fluorwasserstoff, Z-3,3,3-Trifluor-1-chlorpropen, Z-1,3,3,3-Tetrafluorpropen und eine oder mehrere andere (Hydro)Halogenkohlenstoff-Verbindungen mit 1 bis 3 Kohlenstoffatomen.

7. Zusammensetzung nach einem der Ansprüche 1 bis 6, umfassend Fluorwasserstoff, Z-3,3,3-Trifluor-1-chlorpropen, 1,1,1,3,3-Pentafluorpropan und eine oder mehrere andere (Hydro)Halogenkohlenstoff-Verbindungen mit 1 bis 3 Kohlenstoffatomen.

8. Zusammensetzung nach einem der Ansprüche 1 bis 7, welche eine ternäre Mischung ist, und vorzugsweise eine Mischung ist von Fluorwasserstoff, Z-3,3,3-Trifluor-1-chlorpropen und E-3,3,3-Trifluor-1-chlorpropen.

9. Zusammensetzung nach einem der Ansprüche 1 bis 7, welche eine quaternäre Mischung ist, und vorzugsweise eine Mischung ist:
- von Fluorwasserstoff, Z-3,3,3-Trifluor-1-chlorpropen, E-3,3,3-Trifluor-1-chlorpropen und 1,1,1,3,3-Pentafluorpropan; oder
- von Fluorwasserstoff, Z-3,3,3-Trifluor-1-chlorpropen, E-1,3,3,3-Tetrafluorpropen und 1,1,1,3,3-Pentafluorpropan; oder
- von Fluorwasserstoff, Z-3,3,3-Trifluor-1-chlorpropen, Z-1,3,3,3-Tetrafluorpropen und 1,1,1,3,3-Pentafluorpropan.

10. Zusammensetzung nach einem der Ansprüche 1 bis 7, welche eine quinquenäre Mischung ist, und vorzugsweise eine Mischung ist:
- von Fluorwasserstoff, Z-3,3,3-Trifluor-1-chlorpropen, E-3,3,3-Trifluor-1-chlorpropen, Z-1,3,3,3-Tetrafluorpropen und 1,1,1,3,3-Pentafluorpropan; oder
- von Fluorwasserstoff, Z-3,3,3-Trifluor-1-chlorpropen, E-3,3,3-Trifluor-1-chlorpropen, E-1,3,3,3-Tetrafluorpropen und 1,1,1,3,3-Pentafluorpropan; oder
- von Fluorwasserstoff, Z-3,3,3-Trifluor-1-chlorpropen, Z-1,3,3,3-Tetrafluorpropen, E-1,3,3,3-Tetrafluorpropen und 1,1,1,3,3-Pentafluorpropan.

11. Zusammensetzung nach einem der Ansprüche 1 bis 7, welche eine senäre Mischung ist, und vorzugsweise eine Mischung ist:
- von Fluorwasserstoff, Z-3,3,3-Trifluor-1-chlorpropen, E-3,3,3-Trifluor-1-chlorpropen, Z-1,3,3,3-Tetrafluorpropen, E-1,3,3,3-Tetrafluorpropen und 1,1,1,3,3-Pentafluorpropan.

12. Zusammensetzung nach einem der Ansprüche 1 bis 11, welche heteroazeotrop oder quasi-heteroazeotrop ist.

13. Zusammensetzung nach einem der Ansprüche 1 bis 12, welche von 1 bis 85 Gew.-%, vorzugsweise von 1 bis 80 Gew.-%, stärker bevorzugt von 5 bis 80 Gew.-% und besonders stark bevorzugt von 5 bis 75 Gew.-% Fluorwasserstoff; und/oder von 15 bis 99 Gew.-%, vorzugsweise von 20 bis 99 Gew.-%, stärker bevorzugt von 20 bis 95 Gew.-% und besonders stark bevorzugt von 25 bis 95 Gew.-% (Hydro)Halogenkohlenstoff-Verbindungen mit 1 bis 3 Kohlenstoffatomen umfasst.

14. Zusammensetzung nach einem der Ansprüche 1 bis 13, mit einem Kochpunkt von 0 bis 40 °C bei einem Druck von 0,5 bis 9 bar absolut.

15. Verfahren zur Herstellung einer Haupt-(Hydro)Halogenkohlenstoff-Verbindung, umfassend:
- die Bildung einer Mischung von Verbindungen, umfassend Fluorwasserstoff, Z-3,3,3-Trifluor-1-chlorpropen und eine oder mehrere andere (Hydro)Halogenkohlenstoff-Verbindungen;
- die Destillation dieser Mischung, die es gestattet, einerseits eine azeotrope Zusammensetzung nach einem der Ansprüche 1 bis 14 und andererseits mindestens eine der Verbindungen der Mischung zu sammeln.

16. Verfahren nach Anspruch 15, wobei die Destillation gestattet, einerseits eine azeotrope Zusammensetzung nach einem der Ansprüche 1 bis 14 und andererseits Fluorwasserstoff; oder auch einerseits eine azeotrope Zusammensetzung nach einem der Ansprüche 1 bis 14 und andererseits E-3,3,3-Trifluor-1-chlorpropen zu sammeln.

17. Verfahren nach Anspruch 15 oder 16, welches ein Verfahren zur Herstellung von 3,3,3-Trifluor-1-chlorpropen und vorzugsweise E-3,3,3-Trifluor-1-chlorpropen ist.

18. Verfahren nach einem der Ansprüche 15 bis 17, wobei die Mischung von Verbindungen nach einem Schritt der Fluorierung erhalten wird, der das Umsetzen einer chlorierten Verbindung mit Fluorwasserstoff umfasst.

19. Verfahren nach einem der Ansprüche 15 bis 18, wobei die gesammelte azeotrope Zusammensetzung vorzugsweise durch Dekantieren in zwei nichtmischbare flüssige Fraktionen getrennt wird, nämlich eine an Fluorwasserstoff reiche Fraktion und eine an Fluorwasserstoff arme Fraktion, wobei die an Fluorwasserstoff reiche Fraktion einen höheren Anteil an Fluorwasserstoff enthält als die an Fluorwasserstoff arme Fraktion; und wobei die an Fluorwasserstoff reiche Fraktion gegebenenfalls zum Fluorierungsschritt zurückgeführt wird.

20. Verfahren nach einem der Ansprüche 15 bis 19, wobei die chlorierte Verbindung 1,1,1,3,3-Pentachlorpropan oder 1,1,3,3-Tetrachlorpropen ist.

## Claims

1. Azeotropic or quasi-azeotropic composition comprising hydrogen fluoride, Z-3,3,3-trifluoro-1-chloropropene and one or more (hydro)halocarbon compounds comprising from 1 to 3 carbon atoms.

2. Composition according to Claim 1, in which the (hydro)halocarbon compound(s) comprise three carbon atoms, and are preferably chosen from propanes and propenes that are partially or totally substituted with halogens.

3. Composition according to Claim 1 or 2, in which the (hydro)halocarbon compound(s) are chosen from tetrachlorofluoropropanes, trichlorodifluoropropanes, dichlorotrifluoropropanes, chlorotetrafluoropropanes, pentafluoropropanes, dichlorodifluoropropenes, trichlorofluoropropenes, chlorotrifluoropropenes and tetrafluoropropenes.

4. Composition according to one of Claims 1 to 3, comprising hydrogen fluoride, Z-3,3,3-trifluoro-1-chloropropene, E-3,3,3-trifluoro-1-chloropropene and one or more other (hydro)halocarbon compounds comprising from 1 to 3 carbon atoms.

5. Composition according to one of Claims 1 to 4, comprising hydrogen fluoride, Z-3,3,3-trifluoro-1-chloropropene, E-1,3,3,3-tetrafluoropropene and one or more other (hydro)halocarbon compounds comprising from 1 to 3 carbon atoms.

6. Composition according to one of Claims 1 to 5, comprising hydrogen fluoride, Z-3,3,3-trifluoro-1-chloropropene, Z-1,3,3,3-tetrafluoropropene and one or more other (hydro)halocarbon compounds comprising from 1 to 3 carbon atoms.

7. Composition according to one of Claims 1 to 6, comprising hydrogen fluoride, Z-3,3,3-trifluoro-1-chloropropene, 1,1,1,3,3-pentafluoropropane and one or more other (hydro)halocarbon compounds comprising from 1 to 3 carbon atoms.

8. Composition according to one of Claims 1 to 7, which is a ternary mixture, and is preferably a mixture of hydrogen fluoride, Z-3,3,3-trifluoro-1-chloropropene and E-3,3,3-trifluoro-1-chloropropene.

9. Composition according to one of Claims 1 to 7, which is a quaternary mixture, and is preferably a mixture of:
- hydrogen fluoride, Z-3,3,3-trifluoro-1-chloropropene, E-3,3,3-trifluoro-1-chloropropene and 1,1,1,3,3-pentafluoropropane; or
- hydrogen fluoride, Z-3,3,3-trifluoro-1-chloropropene, E-1,3,3,3-tetrafluoropropene and 1,1,1,3,3-pentafluoropropane; or
- hydrogen fluoride, Z-3,3,3-trifluoro-1-chloropropene, Z-1,3,3,3-tetrafluoropropene and 1, 1, 1, 3, 3-pentafluoropropane.

10. Composition according to one of Claims 1 to 7, which is a quinternary mixture, and is preferably a mixture of:
- hydrogen fluoride, Z-3,3,3-trifluoro-1-chloropropene, E-3,3,3-trifluoro-1-chloropropene, Z-1,3,3,3-tetrafluoropropene and 1,1,1,3,3-pentafluoropropane; or
- hydrogen fluoride, Z-3,3,3-trifluoro-1-chloropropene, E-3,3,3-trifluoro-1-chloropropene, E-1,3,3,3-tetrafluoropropene and 1,1,1,3,3-pentafluoropropane; or
- hydrogen fluoride, Z-3,3,3-trifluoro-1-chloropropene, Z-1,3,3,3-tetrafluoropropene, E-1,3,3,3-tetrafluoropropene and 1,1,1,3,3-pentafluoropropane.

11. Composition according to one of Claims 1 to 7, which is a senary mixture, and is preferably a mixture of:
- hydrogen fluoride, Z-3,3,3-trifluoro-1-chloropropene, E-3,3,3-trifluoro-1-chloropropene, Z-1,3,3,3-tetrafluoropropene, E-1,3,3,3-tetrafluoropropene and 1,1,1,3,3-pentafluoropropane.

12. Composition according to one of Claims 1 to 11, which is hetero-azeotropic or quasi-hetero-azeotropic.

13. Composition according to one of Claims 1 to 12, which comprises from 1% to 85% by weight, preferably from 1% to 80% by weight, more particularly preferably from 5% to 80% by weight and most particularly preferably from 5% to 75% by weight of hydrogen fluoride; and/or from 15% to 99% by weight, preferably from 20% to 99% by weight, more particularly preferably from 20% to 95% by weight and most particularly preferably from 25% to 95% by weight of (hydro)halocarbon compounds comprising from 1 to 3 carbon atoms.

14. Composition according to one of Claims 1 to 13, which has a boiling point of 0 to 40°C for a pressure from 0.5 to 9 bar absolute.

15. Process for producing a main (hydro)halocarbon compound, comprising:
- the formation of a mixture of compounds comprising hydrogen fluoride, Z-3,3,3-trifluoro-1-chloropropene and one or more other (hydro)halocarbon compounds;
- distillation of this mixture making it possible to collect, firstly, an azeotropic composition according to one of Claims 1 to 14, and, secondly, at least one of the compounds of the mixture.

16. Process according to Claim 15, in which distillation makes it possible to collect, firstly, an azeotropic composition according to one of Claims 1 to 14, and, secondly, hydrogen fluoride; or alternatively, firstly, an azeotropic composition according to one of Claims 1 to 14, and, secondly, E-3,3,3-trifluoro-1-chloropropene.

17. Process according to Claim 15 or 16, which is a process for producing 3,3,3-trifluoro-1-chloropropene, and preferably E-3,3,3-trifluoro-1-chloropropene.

18. Process according to one of Claims 15 to 17, in which the mixture of compounds is obtained after a fluorination step, comprising the reaction of a chloro compound with hydrogen fluoride.

19. Process according to one of Claims 15 to 18, in which the azeotropic composition collected is separated, preferably by decantation, into two immiscible liquid fractions, namely a fraction rich in hydrogen fluoride and a fraction poor in hydrogen fluoride, the fraction rich in hydrogen fluoride containing a higher proportion of hydrogen fluoride than the fraction poor in hydrogen fluoride; and the fraction rich in hydrogen fluoride being, where appropriate, recycled into the fluorination step.

20. Process according to one of Claims 15 to 19, in which the chloro compound is 1,1,1,3,3-pentachloropropane or 1,1,3,3-tetrachloropropene.
